# EUROPEAN PATENT APPLICATION

(11) **EP 3 330 286 A1**
(43) Date of publication of application: **06.06.2018**
(21) Application number: 16382579.7
(22) Date of filing: 30.11.2016
(51) Int. Cl.: C07K 16/12, G01N 33/569

(54) **METHODS AND COMPOSITIONS FOR TUBERCULOSIS DIAGNOSIS**

(71) Applicant: CZ Veterinaria S.A, 36410 Porrino-Pontevedra (ES)
(72) Inventor: DOMÍNGUEZ RODRÍGUEZ, Mercedes, E-28231 Las Rozas-Madrid (ES); INFANTES LORENZO, José Antonio, E-28020 Madrid (ES); MORENO IRUELA, Inmaculada, E-28850 Torrejón de Ardoz-Madrid (ES); DOMÍNGUEZ RODRÍGUEZ, Lucas, E-28230 Las Rozas-Madrid (ES); BEZOS GARRIDO, Javier, E-28232 Las Rozas-Madrid (ES); CASAL COMENDADOR, Carmen, E-24009 León (ES); LIANDRIS, Emmanouil, 2440 Geel (BE); ROMERO MARTÍNEZ, Beatriz, E-28430 Alpedrete-Madrid (ES); DE JUAN FERRÉ, Lucía, E-28760 Tres Cantos-Madrid (ES); ROY CORDERO, Álvaro, E-28029 Madrid (ES); GORTÁZAR SCHMIDT, Ramón Christian, E-13005 Ciudad Real (ES); RISALDE MOYA, María de los Ángeles, E-14011 Córdoba (ES); BALSEIRO MORALES, Ana María, E-24191 Villabalter-León (ES); JUSTE JORDÁN, Ramón A., E-48180 Loiu-Bizkaia (ES)
(74) Representative: ABG Patentes, S.L.

(57) **Abstract**

The present invention relates to the field of veterinary diagnosis and, more in particular, to immunogenic compositions and methods with application in the diagnosis of tuberculosis, particularly bovine tuberculosis.

## Description

### TECHNICAL FIELD

The present invention relates to the field of veterinary diagnosis and, more in particular, to compositions and methods with application in the diagnosis of tuberculosis.

### BACKGROUND ART

Bovine tuberculosis is a chronic disease affecting a wide range of hosts (including humans), for which eradication is sought and that is subject to compulsory declaration in Europe. The current eradication program is based on the diagnosis and slaughter of animals that test positive in official diagnostic tests (intradermal tuberculin (IDTB) test and IFN-γ detection). Despite the effort being made in recent years following this protocol of action, there are some countries where eradication of this disease has still not been achieved.

The antigenic substance used for conducting official diagnostic tests is bovine tuberculin obtained by means of heat inactivation, filtration and precipitation of *Mycobacterium bovis* cultures, giving rise to a product referred to as "purified protein derivative" (PPDb). However, it is difficult to standardize the qualitative, quantitative and functional aspects of the end product. The presence in PPDb of interspecific antigens causes the occurrence of false positives as a result of cross-reactivity with environmental mycobacteria or vaccination against other mycobacteria. For this reason and to prevent it from interfering with diagnosis, countries applying bovine tuberculosis eradication programs prohibit vaccinating these animals (Council Directive 78/52/EEC) against diseases caused by mycobacteria, such as paratuberculosis, for example, in turn causing new economic losses derived from this disease (mainly reduced meat yield and milk production in animals). On the other hand, there is the problem of false negative results, which are particularly abundant in young animals and immunocompromised subjects (such as females in peripartum period and animals in advanced stages of infection). In these cases, the delayed hypersensitivity response is not efficient, leading to an error in the identification of infected animals, allowing them to stay with the herd and constituting a source of infection for other susceptible subjects.

Different approaches in the art have been attempted in order to improve the tests for the diagnosis of bovine tuberculosis. Monoclonal antibodies recognizing antigens MPB70 and MPB83 of *Mycobacterium bovis* have been described (Lyashchenko KP et al. 2001 Scand J Immunol 498-502). Monoclonal antibodies produced by hybridoma cell lines against purified MPB70 protein have been obtained in the article (Haga S et al. 1992 Hybridoma 11(4): 483-492). Monoclonal antibodies recognizing PPDb have been obtained and assayed in ELISA for their efficiency in the diagnosis of tuberculosis (Tolabaevich K et al. 2003 Veterinarski Arhiv 73(2): 81-93). Recombinant MPB70 and MPB83 proteins have been used as well as capture antigens in ELISA for the diagnosis of bovine tuberculosis (Marassi CD et al. 2011 Acta Tropica 118: 101-104). Methods for the isolation and characterization of *M. bovis* antigens are known in the art (Fifis T et al. 1994 Vet Microbiol 40: 65-81). Antibodies for mycobacterial antigen A60 have been obtained in the art and used for detection of tuberculosis infection (Yari Sh et al. 2011 J Microbiol Methods 87: 184-188). The effect of the inoculation site of PPDb in connection with the diagnosis of tuberculosis has been studied (Casal C et al. 2015 Prev Vet Med 121 (1-2): 86-92).

However, there is still needed in the art a standardized and reproducible PPDb, along with diagnostic techniques with a higher sensitivity and specificity, as an essential tool for the eradication of tuberculosis in cattle.

### BRIEF SUMMARY OF THE INVENTION

The inventors have found that the SIM-377.18.2.1 hybridoma, hereinafter SIM 18.2, secretes an antibody which specifically recognizes an epitope present in MPB70 and MPB83 proteins of PPDb. The immunopurified product with said antibody can be used replacing the PPDb protein complex in the diagnosis of bovine tuberculosis and showing better results. Furthermore, since the immunopurified product obtained by the inventors (p22) is a qualitatively, quantitatively and functionally defined compound, standardization among batches will be a considerable improvement during the production and use thereof when compared with a product as complex as PPDb. Particularly, the immunopurified product of the invention differentiates from PPDb in the number of proteins comprised by said product (116 proteins in p22 *versus* more than 600 proteins in PPDb) as well as in the proportion of the combination of MPB70 and MPB83 proteins (at least 32% in p22 *versus* 10% or less in PPDb). Based on the p22 product, the inventors have developed diagnostic methods following the IDTB test, the IFN-γ detection test and the detection of specific antibodies in serum.

Thus, in a first aspect, the present invention relates to an immunogenic composition comprising antigens from bacteria of the *Mycobacterium tuberculosis* complex, wherein said composition is obtainable by immunopurification from a bovine tuberculin purified protein derivative (PPDb) by means of an antibody or an antigen-binding fragment thereof, wherein said antibody comprises complementarity-determining regions (CDRs) that comprise amino acid sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.

In a further aspect, the invention relates to a method for the *in vitro* diagnosis of tuberculosis in a subject comprising
(i) contacting a sample, preferably a blood sample, from said subject with the immunogenic composition as above,
(ii) determining whether complexes between the antigens of the immunogenic composition as above and antibodies specific for said antigens in the sample are formed,
   wherein the formation of such complexes between the antigens of the immunogenic composition as above and antibodies specific for said antigens in the sample is indicative of a diagnosis of tuberculosis.

In a further aspect, the invention relates to a method for the *in vitro* diagnosis of tuberculosis in a subject comprising
(i) contacting a sample from said subject with the immunogenic composition as above and,
(ii) determining the levels of interferon-γ (IFN- y) produced as a consequence of step (i),
   wherein if IFN-γ levels are increased with respect to a reference value, then the subject is diagnosed with tuberculosis.

In a further aspect, the invention relates to a method for determining the immunoreactivity to bacteria of the *Mycobacterium tuberculosis* complex in a subject that comprises
(i) inoculating intradermically the immunogenic composition as above, and
(i) determining the skinfold thickness in the inoculation area after the intradermal inoculation,
   wherein if the increase in the skinfold thickness is at least 2 mm greater than a reference value, then the subject is immunoreactive to bacteria of the *Mycobacterium tuberculosis* complex.

In a further aspect, the invention relates to the use of the immunogenic composition as above in the diagnosis of tuberculosis.

In a further aspect, the invention relates to an antibody or an antigen-binding fragment thereof, wherein said antibody comprises complementarity-determining regions (CDRs) comprising sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.

### BRIEF DESCRIPTION OF THE FIGURES

**Figure 1** shows the SDS-PAGE electrophoresis (12.5%) of PPDb and SIM 18.2 monoclonal antibody (MAb) with Coomassie blue staining: a PPDb sample (Panel A, lane 2) and a SIM 18.2 MAb sample (panel B, lane 2) were subjected to electrophoresis under reducing conditions. Subsequently, the gels were stained with Coomassie blue. Lane 1 in both panels corresponds to molecular weight markers.
**Figure 2** shows the locations of the parts of the neck selected for inoculating the purified fractions in goats.
**Figure 3** shows the western blot (WB) analysis of the reactivity of SIM 18.2 MAb against PPDb. A PPDb sample was subjected to SDS-PAGE electrophoresis under reducing condition (lane 2) and non-reducing condition (lane 3) followed by electrotransfer and WB development with the SIM 18.2 anti-p22 MAb. Lane 1 corresponds to molecular weight marker.
**Figure 4** shows the SDS-PAGE electrophoresis (12.5%) of 6 µg of P22 and subsequent staining with Coomassie blue.
**Figure 5** shows the graphical representation of the optical densities of the serum reactivity of 4 goats with respect to different *M. bovis* antigens.
**Figure 6** shows an agarose gel electrophoresis of V_{H} and V_{L} regions amplified by PCR in the CDR characterization of SIM18.2 monoclonal antibody.
**Figure 7** shows some examples of the cloning verification by colony PCR of V_{H} and V_{L} regions from hybridoma SIM377_18.2.1 cloned into pGEM-T easy vector.

### DETAILED DESCRIPTION OF THE INVENTION

The authors of the present invention have obtained a monoclonal antibody (i.e. SIM 18.2) which specifically recognizes some epitopes of proteins of PPDb, including MPB70 and MPB83. Development of the monoclonal antibody SIM18.2 is shown in Example 1, and further detailed in Example 2. Following immunopurification from PPDb based on SIM18.2 MAb, the inventors have obtained an immunogenic product (i.e. p22). Obtention and characterization of the immunogenic product of the invention is shown in Example 1. Diagnostic applications of the immunogenic composition of the invention have been tested *in vivo* by means of the IDTB test, and *in vitro* by means of IFN-γ detection test or by detection of immunocomplexes formed between antigens of p22 and specific antibodies in a sample from the subject under diagnosis (see Example 1). The results show that improved specificity and sensibility values are obtained when diagnosis is based on the p22 composition obtained by the inventors when compared to conventional diagnosis based on PPDb.

### Definitions

The terms "antibody," "immunoglobulin," and the like terms refer to a polypeptide substantially encoded by an immunoglobulin gene or immunoglobulin genes, or fragments thereof, which specifically bind and recognize an analyte (antigen). The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. An exemplary immunoglobulin (antibody) structural unit is composed of two pairs of polypeptide chains, each pair having one "light" (about 25 kD) and one "heavy" chain (about 50-70 kD). The N-terminus of each chain defines a variable region primarily responsible for antigen recognition. The terms variable light chain (VL) and variable heavy chain (VH) refer to these light and heavy chains respectively. The C-terminal ends of each heavy chain are disulfide bonded together, and form the constant region of the antibody. Depending on the amino acid sequence of the constant domain of their heavy chains, antibodies can be assigned to different "classes". There are five-major classes of antibodies: IgA, IgD, IgE, IgG, and IgM, and several of these may be further divided into "subclasses" (isotypes), e.g., in mouse IgG1, IgG2a, IgG2b, IgG3. Full-length immunoglobulin "light chains" (of about 25 kDa) comprise a variable region at the NH2-terminus and a kappa or lambda constant region at the COOH-terminus. Full-length immunoglobulin "heavy chains" (of about 50 kDa) similarly comprise a variable region and one of the aforementioned heavy chain constant regions or classes, e.g., gamma. The subunit structures and three-dimensional configurations of different classes of immunoglobulins are well known. The term "antibody" includes, for example, polyclonal antibodies, monoclonal antibodies, Fab and single chain Fv (scFv) fragments thereof, bispecific antibodies, heteroconjugates, human and humanized antibodies. Such antibodies may be produced in a variety of ways, including hybridoma cultures, recombinant expression in bacteria or mammalian cell cultures, and recombinant expression in transgenic animals. Also antibodies can be produced by selecting a sequence from a library of sequences expressed in display systems such as filamentous phage, bacterial, yeast or ribosome. There is abundant guidance in the literature for selecting a particular production methodology, e.g., Chadd and Chamow, Curr. Opin. Biotechnol., 12:188-194 (2001). The choice of manufacturing methodology depends on several factors including the antibody structure desired, the importance of carbohydrate moieties on the antibodies, ease of culturing and purification, and cost. Many different antibody structures may be generated using standard expression technology, including full-length antibodies, antibody fragments, such as Fab and Fv fragments, as well as chimeric antibodies comprising components from different species. Antibody fragments of small size, such as Fab and Fv fragments, may be generated in a bacterial expression system.

The expression "antibody specific for an antigen", as used herein, relates to an antibody that is generated or obtained against a particular antigen.

The term "antigen", as used herein, relates to a molecule that induces an immune response in the body. Therefore, an antigen can be used for generating an antibody that recognizes it and binds specifically to it. As the person skilled in the art will understand, antigens are detectable as a result of their capability of being specifically recognized by an antibody.

The term "bacteria", as used herein, relates to a large domain of prokaryotic microorganisms showing different morphologies (including spherical, rod-shaped, curved-rods, spiral-shaped, and coiled) and typically 0.5-5.0 micrometres in length.

The term "bovine", as used herein, relates to the family *Bovidae,* subfamily *Bovinae,* including tribe *Boselaphini* (including genus *Tetracerus* and *Boselaphus*), tribe *Bovini* (including genus *Bubalus, Bos, Pseudoryx, Syncerus* and *Bison),* and tribe *Strepsicerotini* (including genus *Tragelaphus* and *Taurotragus*). In particular, this term includes cows and cattle (including genus *Bos,* species *Bos taurus* or domestic cattle).

The term "bovine tuberculin purified protein derivative", abbreviated as PPDb, as used in the present invention, and also known as "tuberculin purified protein derivative, bovine", relates to a preparation obtained from the heat-treated products of growth and lysis of *Mycobacterium bovis* capable of revealing a delayed hypersensitivity in an animal sensitized to micro-organisms of the same species. PPDb is used in the art as a diagnostic agent causing mild, delayed allergic reaction in patients infected with tuberculosis or who have had a past infection. PPDb is obtained from the water-soluble fractions prepared by heating in free-flowing steam and subsequently filtering cultures of *M. bovis* grown in a liquid synthetic medium. The active fraction of the filtrate, consisting mainly of protein, is isolated by precipitation, washed and redissolved. An antimicrobial preservative that does not give rise to false positive reactions, such as phenol, may be added. In a preferred embodiment the PPDb is obtained from *M*.*bovis* strain AN-5, preferably at 25.000 IU/ml.

The term "complementarity-determining region" or "CDR", as used herein, relates to the restricted regions within the variable regions of intact antibodies or antibody fragments that bind to antigenic determinants, i.e. complement an antigen's shape. The CDRs are the most variable part of an antibody and contribute to the diversity of these molecules, allowing the antibody to recognize a vast repertoire of antigens. Thus, the CDRs determine the affinity and specificity of an antibody for specific antigens.

The term "diagnosis", as used herein, relates to both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. In particular, the term "diagnosis of tuberculosis" or, more particularly "diagnosis of bovine tuberculosis", relates to the capacity to identify or detect the presence of tuberculosis, more particularly bovine tuberculosis, in a subject. This diagnosis, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly diagnosing at least 60%, at least 70%, at least 80%, or at least 90% of the subjects of a determined group or population analyzed.

The term "immunoassay", as used herein, relates to a test or assay that measures the presence and/or the concentration of molecule in a solution by using an antibody recognizing a particular antigen, or by using an antigen able to detect the presence of an antibody. The molecule to be detected by the immunoassay can be referred to as an analyte.

The term "immunogenic composition", as used herein, relates to a composition that elicits an immune response in a subject that produces antibodies or cell-mediated immune responses against a specific immunogen.

The term "immunopurification", as used herein, relates to the use of immunological techniques to purify proteins such as antigens and includes, without limitation, immunoprecipitation, affinity chromatography, and immunoaffinity purification. In particular, immunoprecipitation relates to precipitate a protein (i.e. antigen) out of solution using an antibody that specifically binds to that particular protein.

The term "immunoreactivity", as used herein, relates to the presence or level of binding of an antibody or antibodies in a sample to one or more target antigens, for example, antigens of bacterium of the *Mycobacterium tuberculosis* complex.

The term *"Mycobacterium bovis",* as used herein, relates to a Gram positive, acid-fast bacterium in the *Mycobacterium tuberculosis* complex of the family *Mycobacteriaceae.* It is the causative agent of tuberculosis in cattle (bovine tuberculosis).

The term *"Mycobacterium tuberculosis* complex", as used herein, relates to a genetically related group of *Mycobacterium* species that can cause tuberculosis in humans or other living beings, and includes *Mycobacterium tuberculosis, Mycobacterium africanum, Mycobacterium bovis,* particularly the *Bacillus Calmette-Guérin* strain, *Mycobacterium microti, Mycobacterium canetti, Mycobacterium caprae, Mycobacterium pinnipedii, Mycobacterium suricattae,* and *Mycobacterium mungi.*

The term "interferon gamma", as used herein, also known as "IFN-γ" or "IFNγ" relates to a dimerized soluble cytokine representing the only member of the type II class of interferons. It has a critical role for innate and adaptive immunity against viral, some bacterial and protozoal infections. IFNγ is an important activator of macrophages and inducer of Class II major histocompatibility complex (MHC) molecule expression.

The term "sample" as used herein, relates to a biofluid sample that can be obtained from a subject. In a preferred embodiment of the invention, the biofluid is any fluid of the body comprising antibodies. In a more preferred embodiment of the invention, the biofluid is selected from blood, serum, plasma, peritoneal fluid and cerebrospinal fluid. In a more preferred embodiment of the invention, the biofluid is selected from blood (such as peripheral blood), serum or plasma. In a more preferred embodiment, the sample is blood. In humans, the blood sample is typically extracted by means of puncturing an artery or vein, normally a vein from the inner part of the elbow or from the back of the hand, the blood sample being collected in an air-tight vial or syringe. A capillary puncture normally on the heel or on the distal phalanxes of fingers can be performed for analysis by means of a micromethod. Blood extraction in domestic small ruminants (sheep and goats) and wild cervids is performed from jugular vein in the neck or the coccygeal vessels in proximal segment of the tail and only requires physical restrain if performed by a skilled person. In cattle, blood samples are collected from the tail vein and also requires a safe physical restrain. In some cases, blood samples can be also collected form the jugular or even the mammary veins. The using of vacutainers for venipuncture is recommended to ensure an sterile and fast blood extraction. In other species different places might be chosen including the inner corner of the eye. Serum can be obtained from the complete blood sample and in the absence of anticoagulant by leaving the sample to settle for 10 minutes so that it coagulates and by subsequently centrifuging it at 1200xg for 10 minutes for the purpose of separating the cells (precipitate) from the serum (supernatant). In turn, to obtain the plasma sample the complete blood is mixed with an anticoagulant and is centrifuged at 1200xg for 20 minutes. The precipitate of said centrifugation corresponds to the formed elements, and the supernatant corresponds to the plasma. The serum or the plasma obtained can be transferred to a storage tube for sample analysis by means of the methods of the invention.

The term "subject" or "individual" or "animal" or "patient" or "mammal," is meant any subject, particularly a mammalian subject, for whom diagnosis, prognosis, or therapy is desired. Mammalian subjects include humans, domestic animals, farm animals, and zoo, sports, or pet animals such as dogs, cats, guinea pigs, rabbits, rats, mice, horses, cattle, cows, sheep, goats, and so on. In a preferred embodiment of the invention, the subject is a mammal, particularly a mammal susceptible of being infected by a bacteria of the *Mycobacterium tuberculosis* complex, more particularly a mammal susceptible of being infected by *Mycobacterium bovis, Mycobacterium caprae,* or *Mycobacterium tuberculosis,* more particularly by *Mycobacterium bovis,* even more particularly a mammal selected from the group consisting of a red deer *(Cervus elaphus*), a wild boar (*Sus scrofa*), a goat (*Capra aegagrus hircus),* a cow *(Bos taurus)* and a sheep (*Ovis aries*).

The term "bovine tuberculosis", as used herein, relates to a chronic bacterial disease of cattle that occasionally affects other species of mammals, including humans, and is the result of an infection by any mycobacteria of the *Mycobacterium tuberculosis complex. Mycobacterium bovis* causative agent of bovine tuberculosis, although having cattle as the primary hosts, other domesticated and wild mammals can also be infected. Known maintenance hosts include brush-tailed opossums (and possibly ferrets) in New Zealand, badgers in the United Kingdom and Ireland, bison and elk in Canada, and kudu and African buffalo in southern Africa. White-tailed deer in the United States (Michigan) have been classified as maintenance hosts; however, some authors now believe this species may be a spillover host that maintains the organism only when its population density is high. Species reported to be spillover hosts include sheep, goats, horses, pigs, dogs, cats, ferrets, camels, Ilamas, many species of wild ruminants including deer and elk; elephants, rhinoceroses, foxes, coyotes, mink, primates, opossums, otters, seals, sea lions, hares, raccoons, bears, warthogs, large cats (including lions, tigers, leopards, cheetahs and lynx) and several species of rodents. Most mammals may be susceptible. Birds are generally thought to be resistant to *M. bovis* infection. In a particular embodiment of the invention, the tuberculosis is bovine tuberculosis.

### Immunogenic composition of the invention

The inventors obtained an immunogenic composition (p22) by immunopurification from PPDb using monoclonal antibody SIM 18.2 (see Example 1). The protein composition of p22 was determined (see Table 1) and CDRs of the monoclonal antibody SIM 18.2 were sequenced (see Example 2).

Thus, in a first aspect, the invention relates to an immunogenic composition comprising antigens from bacteria of the *Mycobacterium tuberculosis* complex, wherein said composition is obtainable by immunopurification from a PPDb by means of an antibody or an antigen-binding fragment thereof, wherein said antibody comprises complementarity-determining regions (CDRs) that comprise amino acid sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.

In a particular embodiment, the immunogenic composition of the invention comprises antigens from bacteria of the *Mycobacterium tuberculosis* complex that are selected from the group consisting of MPB70, MPB83, molecular chaperone GroEL, MPB64, 5-methyltetrahydropteroyltriglutamate-homocysteine methyltransferase, ESAT-6-like protein EsxB, elongation factor Tu, ESAT-6, alpha-crystallin and combinations thereof. A description of said antigens is shown in Table 1. In a more particular embodiment, the immunogenic composition of the invention comprises antigens from bacteria of the *Mycobacterium tuberculosis* complex that are selected from the group consisting of MPB70, MPB83 and their combination.

In a particular embodiment, the immunogenic composition of the invention comprises less than 500 proteins, preferably less than 400 proteins, preferably less than 300 proteins, preferably less than 200 proteins, preferably less than 190 proteins, preferably less than 180 proteins, preferably less than 170 proteins, preferably less than 160 proteins, preferably less than 150 proteins, preferably less than 140 proteins, preferably less than 130 proteins, or preferably less than 120 proteins. In a particular embodiment the immunogenic composition of the invention comprises, consists or consists essentially of the proteins of Tables 1 and 2.

In a particular embodiment, the immunogenic composition of the invention comprises a proportion of the combination of antigens MPB70 and MPB83 of at least 15%, preferably of at least 20%, preferably of at least 25%, preferably of at least 26%, preferably of at least 27%, preferably of at least 28%, preferably of at least 29%, or preferably of at least 30%.

"MPB70", as used herein, relates to a mycobacterial antigen which is highly homologous within *Mycobacterium tuberculosis* complex members. MPB70 is encoded as precursor protein with a signal peptide for export through the general secretory pathway. It is a soluble secreted protein cleaved by signal peptidase I. *Mycobacterium bovis* DNA sequence for MPB70 (BCG Tokyo strain) can be accessed at NCBI database under accession number D38230 (GI: 1008918, 1009 bp, version as of 8 February 1999). *Mycobacterium bovis* DNA sequence for MPB70 (BCG Pasteur strain) can be accessed at NCBI database under accession number D38229 (GI: 1008916, 1009 bp, version as of 8 February 1999). *Mycobacterium bovis* protein sequence for MPB70 can be accessed at NCBI database under accession number BAA07403 (GI:1008919, 193 aa, version as of 8 February 1999, corresponding to BCG Tokyo strain). *Mycobacterium tuberculosis* complex protein sequence for MPB70 cell surface protein can be accessed at NCBI database under accession number WP_003414644.1 (GI: 489509783, 193 aa, version as of 25 November 2015).

"MPB83", as used herein, relates to a mycobacterial antigen which is highly homologous within *Mycobacterium tuberculosis* complex members. As MPB70, MPB83 is encoded as precursor protein with a signal peptide for export through the general secretory pathway. However, MPB83 is a glycosylated lipoprotein processed by signal peptidase II and located at the surface. *Mycobacterium bovis* DNA sequence for MPB83 can be accessed at NCBI database under accession number EU683972 (GI: 188523816, 663 bp, version as of 21 May 2008) and under accession number D64165 (GI: 994767, 1107bp, version as of 19 February 2008). *Mycobacterium bovis* protein sequence for MPB83 can be accessed at NCBI database under accession number ACD61707 (GI:188523817, 220 aa, version as of 21 May 2008) and under accession number BAA11027 (GI:994768, 220 aa, version as of 19 February 2008). *Mycobacterium tuberculosis* complex protein sequence for MPB83 cell surface protein can be accessed at NCBI database under accession number WP_003414630.1 (GI: 489509769, 220 aa, version as of 25 November 2015).

"Molecular chaperone GroEL" relates to a molecular chaperone belonging to the chaperonin family, which is found in a large number of bacteria. In order to function properly, GroEL requires the cochaperonin protein complex GroES. GroEL is a dual-ringed tetradecamer, with both the cis and trans rings consisting of seven subunits each. *Mycobacterium tuberculosis* complex protein sequence for GroEL can be accessed at NCBI database under accession number WP_003402236.1 (GI: 489497323, 540 aa, version as of 25 November 2015).

"MPB64" relates to a mycobacterial antigen. *Mycobacterium tuberculosis* complex protein sequence for MPB64 can be accessed at NCBI database under accession number WP_003409954.1 (GI: 489505073, 228 aa, version as of 25 November 2015).

"5-methyltetrahydropteroyltriglutamate-homocysteine methyltransferase" relates to a trasferase enzyme catalyzing a chemical reaction wherein the substrates are 5-methyltetrahydropteroyltri-L-glutamate and L-homocysteine, and wherein the products are tetrahydropteroyltri-L-glutamate and L-methionine. The protein sequence in *Mycobacterium tuberculosis* complex can be accessed at NCBI database under accession number WP_003405914.1 (GI: 489501012, 759 aa, version as of 25 November 2015).

"ESAT-6-like protein EsxB" is also known as CFP-10 or MTSA-10. *Mycobacterium tuberculosis* complex protein sequence for EsxB can be accessed at NCBI database under accession number WP_003399940.1 (GI: 489495023, 100 aa, version as of 25 November 2015).

"Elongation factor Tu", also known as elongation factor thermo unstable, relates to a prokaryotic elongation factor involved in protein synthesis. It helps the aminoacyl-tRNA move onto a free site on the ribosome. In the cytoplasm, EF-Tu binds an aminoacylated, or charged, tRNA molecule. *Mycobacterium tuberculosis* complex protein sequence for elongation factor Tu can be accessed at NCBI database under accession number WP_003403463.1 (GI: 489498552, 396 aa, version as of 25 November 2015).

"ESAT-6" is a virulence factor that triggers cell-mediated immune responses and IFN-γ production during tuberculosis. ESAT-6 is transported across the bacterial envelope by a specialized secretion system with a FSD (FtsK-SpoIIIE domain) membrane protein. *Mycobacterium tuberculosis* complex protein sequence for ESAT-6 can be accessed at NCBI database under accession number WP_003399963.1 (GI: 489495046, 95 aa, version as of 25 November 2015).

"alpha-crystallin" relates to a family of molecular chaperones in *Mycobacterium tuberculosis. Mycobacterium tuberculosis* complex protein sequence for alpha-crystallin can be accessed at NCBI database under accession number WP_003410189.1 (GI 489505308, 144 aa, version as of 25 November 2015).

The immunogenic composition of the invention is obtainable by immunopurification from a bovine tuberculin purified protein derivative (PPDb) by means of an antibody or an antigen-binding fragment thereof. PPDb obtained from *Mycobacterium bovis* is commercially available from CZ Veterinaria (*Mycobacterium bovis,* strain AN-5, 25,000 IU/ml). PPDb can also be obtained by following the standards described in the European Pharmacopoeia as shown herewith in Example 1.

In a preferred embodiment the immunopurification is carried out by affinity chromatograpy, preferably by using HiTrap NHS-activated HP columns conjugated with the MAb.

Immunopurification from PPDb according to the present invention is performed by means of an antibody or an antigen-binding fragment thereof, said antibody comprising complementarity-determining regions (CDRs) that comprise the following amino acid sequences:
- RSSQSIVHSNGNTYLE (CDR-L1) (SEQ ID NO: 1),
- KVSNRFS (CDR-L2) (SEQ ID NO: 2),
- FQGSHVPWT (CDR-L3) (SEQ ID NO: 3),
- GYSFTGYFMN (CDR-H1) (SEQ ID NO: 4),
- RINPYNGDTFYNQKFKG (CDR-H2) (SEQ ID NO: 5), and
- WDGAPTYFFDY (CDR-H3) (SEQ ID NO: 6) or variants thereof.

CDRH1, CDRH2 and CDRH3 correspond to the VH (heavy chain) variable domain, whereas CDRL1, CDRL2 and CDRL3 correspond to the VL (light chain) variable domain of the antibody. The antibody includes any type of known antibody that comprises the six CDRs mentioned above, such as, for example, monoclonal antibodies and genetically engineered antibodies, such as chimeric antibodies, humanized antibodies, primatized antibodies, etc. Antigen-binding fragments of the antibody include, without limitation, Fab (fragment antigen-binding), Fab' (fragment formed by reduction of F(ab')₂ fragments), F(ab')₂ (fragment containing two binding regions joined at the hinge through disulfides), and Fv (smallest fragment containing a complete antigen-binding site) fragments.

In a more particular embodiment, the antibody comprises CDRs within the light chain variable region that comprise amino acid sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), and comprises CDRs within the heavy chain variable region that comprise amino acid sequences GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6).

Variants of any of the CDR sequences above also fall within the scope of the antibody for immunopurification of the immunogenic composition of the invention. As it is used herein, the term "variant" refers to substantially similar sequences. The variants generally have the same biological activity from the qualitative view point as the native sequence, i.e. same antigen binding activity. As a way of example, antigen binding activity may be checked routinely by an indirect ELISA and an antigen internal control to test the title of antibody. Biological activity can be determined, as a way of example, as shown in Example 1, paragraph "2.1. Determination of specific antibodies", provided herewith. A variant of a polypeptide sequence can be a polypeptide sequence comprising the addition, deletion or substitution of one or more amino acids. The variants can differ from the described sequences within framework regions or within the CDRs of any of the heavy or light chains. By way of illustration, the invention includes antibodies, for example, monoclonal antibodies, or fragments thereof with the capacity to bind to PPDb, comprising amino acid sequences having at least approximately 70% sequence identity with the amino acid sequences shown in SEQ ID NO: 1-6, preferably at least 75%, 80%, 85%, 90%, 91%, 92%, 93%, 94%, 95%, 96%, 97%, 98% or 99% sequence identity with the amino acid sequences shown in SEQ ID NO: 1-6. Wherein the variant has a different length that the amino acid sequence according to any of SEQ ID NO: 1-6, in one embodiment the sequence identity can be determined over the whole length of said amino acid sequence according to SEQ ID NO: 1-6, for example, sequence identity of a variant of the amino acid sequence of SEQ ID NO: 1 is determined over the whole length of SEQ ID NO: 1, and so on for variants of the amino acid sequences SEQ ID NO: 2-6. In another embodiment, the sequence identity can be determined over the whole length of the variant amino acid sequence, for example, sequence identity of a variant of the amino acid sequence of SEQ ID NO: 1 is determined over the whole length of said variant of SEQ ID NO: 1, and so on for variants of the amino acid sequences SEQ ID NO: 2-6.

In a particular embodiment, the antibody by means of which the immunogenic composition of the invention is obtainable is an IgG antibody.

In a particular embodiment, the bacteria of the *Mycobacterium tuberculosis* complex whose antigens are comprised by the immunogenic composition used in the methods are *Mycobacterium bovis* bacteria or *Mycobacterium tuberculosis* bacteria, more particularly *Mycobacterium bovis* bacteria.

In a further aspect, the present invention also relates to the use of the immunogenic composition of the invention as described above in the diagnosis of tuberculosis. In a particular embodiment, the use of the immunogenic composition of the invention in the diagnosis or tuberculosis involves intradermal inoculation of said immunogenic composition in the subject to be diagnosed. Subjects to be diagnosed according to the use of the immunogenic composition of the invention include any mammal susceptible of being infected by bacteria of the *Mycobacterium tuberculosis* complex, particularly by *Mycobacterium tuberculosis* or by *Mycobacterium bovis.* In a particular embodiment, the subject is a mammal selected from the group consisting of a red deer (*Cervus elaphus*), a wild boar (*Sus scrofa*), a goat (*Capra aegagrus hircus),* a cow (*Bos taurus)* and a sheep (*Ovis aries*). In a particular embodiment, the tuberculosis is bovine tuberculosis.

In a further aspect, the invention relates to the antibody by means of which the immunogenic composition of the invention is obtainable. Thus, the invention also relates to an antibody or an antigen-binding fragment thereof, wherein said antibody comprises complementarity-determining regions (CDRs) comprising sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof, as described above.

### Tuberculosis diagnostic methods of the invention

The inventors have tested the diagnostic applications of the immunogenic composition p22. Particularly, they have tested the diagnostic value of p22 *in vivo* by means of the IDTB test, and *in vitro* by means of the IFN-γ detection test or by detection of immunocomplexes formed between antigens of p22 and specific antibodies in a sample from the subject under diagnosis (humoral immune response) (see Example 1).

Thus, in another aspect, the present invention relates to a method for the *in vitro* diagnosis of tuberculosis in a subject (first diagnostic method of the invention) comprising
(i) contacting a sample, preferably a blood sample, from said subject with the immunogenic composition of the invention as described above and,
(ii) determining whether complexes between the antigens of the immunogenic composition according to the invention and antibodies specific for said antigens in the sample are formed,
   wherein the formation of such complexes between the antigens of the immunogenic composition according to the invention and antibodies specific for said antigens in the sample is indicative of a diagnosis of tuberculosis.

Thus, in a first step, the first diagnostic method of the invention comprises contacting a sample from a subject whose diagnosis of tuberculosis is to be performed and the immunogenic composition of the invention comprising antigens from bacteria of the *Mycobacterium tuberculosis* complex, as described above.

Samples according to the invention include any kind of sample comprising antibodies and include, without limitation, blood, serum, plasma, peritoneal fluid and cerebrospinal fluid. In a particular embodiment, the sample is selected from plasma, blood and serum.

Conditions suitable for the contacting step of the sample from the subject to be diagnosed and the immunogenic composition of the invention include appropriate incubation time, temperature, sample concentration, etc. and can be determined routinely by the skilled person. In a particular embodiment, the temperature ranges from 4 to 40 °C, in particular from 10 to 39 °C, more particularly from 25 to 39 °C, preferably from 36 to 39 °C, even more preferably at 37 °C. In a particular embodiment, the sample of the subject to be diagnosed of tuberculosis according to the invention is incubated with the immunogenic composition for at least 1 minute, preferably for at least 5 minutes, more preferably for at least 30 minutes, even more preferably for at least 60 minutes. In a particular embodiment, the sample of the subject to be diagnosed of tuberculosis according to the invention may be put into contact with the immunogenic composition without being previously diluted or diluted, wherein dilution is at least 1:2, preferably at least 1:5, more preferably at least 1:10, even more preferably at least 1:100, even more preferably at least 1:200. As the skilled person understands, different buffers are suitable for dilution of the sample. In a particular embodiment, dilution of the sample is performed in phosphate-buffered saline (PBS).

In a second step, the first diagnostic method of the invention comprises determining whether complexes between the antigens of the immunogenic composition according to the invention and antibodies specific for said antigens in the sample are formed. If complexes between the antigens of the immunogenic composition according to the invention and antibodies specific for said antigens in the sample are formed, then the subject is diagnosed of tuberculosis.

In a particular embodiment, determination of the formation of complexes between the antigens of the immunogenic composition of the invention and antibodies specific for said antigens in the sample, if present, is performed by an immunological assay. Immunological assays according to the present invention include any method suitable for determination of antibody levels or antibody titer of antibodies with a desired specificity known by the skilled in the art and include, but are not limited to, immunoassays such as ELISA (Enzyme-Linked Immuno Sorbent Assay), immunostaining, immunochemical assays, immunofluorescence, flow cytometry, Western blot, radioimmunoassays, immunohistochemical assays and immunoprecipitations. By way of non-limiting example, the levels of antibodies specific for the antigens of the immunogenic composition according to the invention can be quantified by means of secondary antibodies with a capacity to specifically bind to said antibodies (or to fragments thereof) and subsequent quantification of the resulting complexes. The antibodies to be employed in these assays can be, for example, polyclonal sera, hybridoma supernatants or monoclonal antibodies, antibody fragments, Fv, Fab, Fab' y F(ab')2, ScFv, diabodies, triabodies, tetrabodies and humanised antibodies. At the same time, the antibodies can be labeled or not. Illustrative, but non-exclusive examples of markers which can be used include radioactive isotopes, enzymes, fluorophores, chemiluminescent reagents, enzymatic substrates or cofactors, enzymatic inhibitors, particles, colorants, etc.

In a particular embodiment, the presence of antibodies specific for the antigens of the immunogenic composition of the invention is determined in a sample from a subject whose diagnosis is intended by means of ELISA or by means of western blot. In a particular embodiment, the ELISA is an indirect ELISA wherein plates are coated with the immunogenic composition according to the invention. Plates are blocked after this coating. The sample of the subject to be diagnosed is then added to the plate.

In a particular embodiment of the first diagnostic method of tuberculosis of the invention, the bacteria of the *Mycobacterium tuberculosis* complex whose antigens are comprised by the immunogenic composition used in the methods are *Mycobacterium caprae, Mycobacterium bovis* or *Mycobacterium tuberculosis* bacteria, more particularly *Mycobacterium bovis.*

In a particular embodiment, the first diagnostic method of tuberculosis of the invention is aimed at the diagnosis of bovine tuberculosis.

In a particular embodiment, the subject to be diagnosed by the first diagnostic method of the invention is a mammal susceptible of being infected by bacteria of the *Mycobacterium tuberculosis* complex. In a particular embodiment, the subject is a mammal susceptible of being infected by *Mycobacterium caprae, Mycobacterium bovis* or *Mycobacterium tuberculosis,* more particularly by *Mycobacterium bovis.* In a preferred embodiment, the subject is a mammal selected from the group consisting of a red deer (*Cervus elaphus*), a wild boar (*Sus scrofa*), a goat (*Capra aegagrus hircus),* a cow (*Bos taurus)* and a sheep (*Ovis aries*).

In a still further aspect, the present invention relates to a method for the *in vitro* diagnosis of tuberculosis in a subject (second diagnostic method of the invention) that comprises
(i) contacting a sample from said subject with the immunogenic composition according to the invention as described above, and
(ii) determining the levels of IFN-γ produced as a consequence of step (i),
   wherein if IFN-γ levels are increased with respect to a reference value, then the subject is diagnosed with tuberculosis.

Thus, in a first step, the second diagnostic method of the invention comprises contacting a sample from the subject to be diagnosed of tuberculosis with the immunogenic composition comprising antigens from bacteria of the *Mycobacterium tuberculosis* complex, as described above.

The step of contacting the sample from the subject to be diagnosed with tuberculosis and the immunogenic composition of the invention, as well as suitable samples, have been described in the context of the first diagnostic method of the invention and incorporated herewith.

In a second step, the second diagnostic method of the invention comprises determining the levels of IFN- γ produced as a consequence of previous first step, wherein if IFN-γ levels are increased with respect to a reference value, then the subject is diagnosed with tuberculosis.

Methods to determine IFN-γ levels are known in the art and include, without limitation, any ELISA assay suitable for IFN-γ determination, as the assay described by Rothel (Rothel JS et al. 1992 Aust Vet J 69(1): 1-4), as well as commercially available kits including, without limitation, Bovigam® TB kit by ThermoFischer Scientific (reference 63320), and Bovine Interferon Gamma ELISA kit by Bio-Rad (reference MCA5638KZZ).

According to the second diagnostic method of the invention, if IFN-γ levels are increased with respect to a reference value, then the subject is diagnosed with tuberculosis. In particular, an increase in the levels of IFN-γ levels with respect to the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" levels. On the other hand, a decrease in the levels of IFN-γ levels with respect to the reference value of at least 0.9-fold, 0.75-fold, 0.2-fold, 0.1-fold, 0.05-fold, 0.025-fold, 0.02-fold, 0.01-fold, 0.005-fold or even less compared with reference value is considered as "decreased" levels. A "lack of change" in the levels of IFN-γ levels with respect to a reference value refers to levels which are substantially unaltered with respect to the reference value. For instance, a lack of change in the levels of IFN-γ levels in the sample under analysis is considered when the levels differ by no more than 0.1%, no more than 0,2%, no more than 0,3%, no more than 0,4%, no more than 0,5%, no more than 0,6%, no more than 0,7%, no more than 0,8%, no more than 0,9%, no more than 1%, no more than 2%, no more than 3%, no more than 4%, no more than 5%, no more than 6%, no more than 7%, no more than 8%, no more than 9%, no more than 10% or no more than the percentage value that is the same as the error associated to the experimental method used in the determination.

The term "reference value" relates to the level of IFN-γ levels in a sample from one or more healthy subjects or from one or more subjects not diagnosed with tuberculosis, particularly not diagnosed with bovine tuberculosis. In a particular embodiment, the reference value is determined in a blood sample.

Once this reference value is established, the level of IFN-γ in a subject whose tuberculosis diagnosis is to be determined can be compared with this reference value, and thus be assigned a "low" or "decreased" level of IFN-γ if it is under this reference value, or a level of "high" or "increased" level of IFN-γ if it is above this reference value.

Once a comparison between the levels of IFN-γ levels in a sample from a subject whose tuberculosis diagnosis is to be determined and the reference value has been made, the second diagnostic method of the invention allows diagnosis of tuberculosis, wherein if IFN-γ levels are increased with respect to a reference value, then the subject is diagnosed with tuberculosis.

In a particular embodiment of the second diagnostic method of tuberculosis of the invention, the bacteria of the *Mycobacterium tuberculosis* complex whose antigens are comprised by the immunogenic composition used in the methods are *Mycobacterium bovis* of *Mycobacterium tuberculosis* bacteria, more particularly *Mycobacterium bovis.*

In a particular embodiment, the second diagnostic method of tuberculosis of the invention is aimed at the diagnosis of bovine tuberculosis.

In a particular embodiment, the subject to be diagnosed by the second diagnostic method of the invention is a mammal susceptible of being infected by bacteria of the *Mycobacterium tuberculosis* complex. In a particular embodiment, the subject is a mammal susceptible of being infected by *Mycobacterium caprae, Mycobacterium bovis* or *Mycobacterium tuberculosis,* more particularly by *Mycobacterium bovis.* In a preferred embodiment, the subject is a mammal selected from the group consisting of a red deer (*Cervus elaphus*), a wild boar (*Sus scrofa*), a goat (*Capra aegagrus hircus*), a cow (*Bos taurus)* and a sheep (*Ovis aries*).

### Immunoreactivity detection method of the invention

The inventors have tested the diagnostic applications of the immunogenic composition p22, more particularly, they have tested the diagnostic value of p22 by means of the intradermal tuberculin (IDTB) test (see Example 1).

Thus, in a further aspect, the invention relates to a method for determining the immunoreactivity to bacteria of the *Mycobacterium tuberculosis* complex in a subject that comprises
(i) inoculating intradermically the immunogenic composition according the invention as described above, and
(ii) determining the skinfold thickness in the inoculation area after the intradermal inoculation,
   wherein if the increase in the skinfold thickness is at least 2 mm greater than a reference value, then the subject is immunoreactive to a bacteria of the *Mycobacterium tuberculosis* complex.

Alternatively, the invention relates to a method for the diagnosis of tuberculosis in a subject that comprises
(i) inoculating intradermically the immunogenic composition according to invention, and
(ii) determining the skinfold thickness in the inoculation area after the intradermal inoculation,
   wherein if the increase in the skinfold thickness is at least 2 mm greater than a reference value, then the subject is diagnosed with tuberculosis.

Thus, the method for determining immunoreactivity involves inoculating intradermically the immunogenic composition of the invention to determine immunoreactivity to bacteria of the *Mycobacterium tuberculosis* complex in a subject. In a particular embodiment, the method determines immunoreactivity to inactivated bacteria of the *Mycobacterium tuberculosis* complex in a subject. Intradermal inoculation or injection involves the inoculation into the dermis, just below the epidermis, and is a routine via of inoculation for sensitivity tests such as tuberculin test. This via of inoculation allows the analysis of the response of a subject to a particular substance, such as the immunogenic composition according to the present invention, since the reaction is closer to the body surface and easily visible. Intradermal inoculation of a substance, particularly of the immunogenic composition of the invention, does not involve any health risk for the subject who is inoculated, since any possible response to the substance occurs immediately under the skin and far away from any vein.

In a subsequent step of the method of the invention for determining immunoreactivity, the skinfold thickness in the inoculation area is determined.

In a particular embodiment, the skinfold thickness in the inoculation area is determined at least one day after the intradermal inoculation of the immunogenic composition of the invention, preferably at least two days after the intradermal inoculation of the immunogenic composition of the invention, more preferably at least three days after the intradermal inoculation of the immunogenic composition of the invention. In a particular preferred embodiment, the skinfold thickness in the inoculation area is determined three days after the intradermal inoculation of the immunogenic composition of the invention.

Methods to determine skinfold thickness in a skin area are routine for the skilled person, especially in the veterinary practice, and include measuring by means of a skinfold-calliper (available from Harpenden, Slimglide or Lange, as a way of example).

The skinfold thickness in the inoculation area is then compared to a reference value. The reference value corresponds to a skinfold thickness determined in the subject whose immunoreactivity to bacteria of the *Mycobacterium tuberculosis* complex is to be determined, wherein said skinfold thickness is determined prior to the inoculation of the immunogenic composition of the invention and is determined in the same body region wherein the immunogenic composition of the invention will be inoculated.

Thus, according to the invention, if the increase in the skinfold thickness is at least 2 mm greater than a reference value, then the subject is immunoreactive to a bacteria of the *Mycobacterium tuberculosis* complex. In a particular embodiment, if the skinfold thickness increase is of 2 mm or more compared to the reference value, then the subject is immunoreactive to a bacteria of the *Mycobacterium tuberculosis* complex. In a particular embodiment, if the skinfold thickness increase is of 4 mm or more compared to the reference value, then the subject is immunoreactive to a bacteria of the *Mycobacterium tuberculosis* complex.

In an alternative embodiment, if the skinfold thickness increase is of 2 mm or more compared to the reference value as defined above after inoculation of the immunogenic composition of the invention and, additionally, said skinfold thickness increase after inoculation of the immunogenic composition to the invention is of 1 mm or more compared to the skinfold thickness increase value after inoculation of PPDa (PPD from *Mycobacterium avium*), then the subject is immunoreactive to a bacteria of the *Mycobacterium tuberculosis* complex. In a particular embodiment, additionally to skinfold thickness determination, the method of the invention for determining immunoreactivity involves determining whether local inflammatory clinical signs are detected, particularly in the inoculation area. In a particular embodiment, the skinfold thickness in the inoculation area and the presence of local inflammatory clinical signs are determined, wherein if the increase in the skinfold thickness is at least 2 mm greater than a reference value according to any of the embodiments above, and wherein if local inflammatory clinical signs are detected, then the subject is immunoreactive to a bacteria of the *Mycobacterium tuberculosis* complex. In a particular embodiment, if the skinfold thickness increase is of 2 mm or more compared to the reference value, and local inflammatory signs are detected, then the subject is immunoreactive to a bacteria of the *Mycobacterium tuberculosis* complex. In a particular embodiment, if the skinfold thickness increase is of 4 mm or more compared the reference value, and local inflammatory signs are detected then the subject is immunoreactive to a bacteria of the *Mycobacterium tuberculosis* complex. In a particular embodiment, if the skinfold thickness increase is of 2 mm or more compared to the reference value, if the skinfold thickness increase is of 1 mm or more compared to the skinfold thickness increase value after inoculation of PPDa, and if local inflammatory signs are detected, then the subject is immunoreactive to a bacteria of the *Mycobacterium tuberculosis* complex

Local inflammation signs include any sign of inflammation occurring in an isolated area of the body, usually in the inoculation area or nearby, which typically becomes red, hot, swollen and sore. In a particular embodiment, local inflammatory clinical signs include oedema (accumulation of watery fluid), exudation (escape of fluid, cells or cellular debris from blood vessels and deposition in or on the tissue), pain (physical unpleasant feeling caused by disease, injury or any factor hurting the body), and inflammatory reaction of the local lymph nodes. Determination of local inflammatory clinical signs as above can be performed by the skilled person following routine procedures. In a way of example, oedema can be determined, if not obvious at a glance, by gently pressing the area with slow, steady pressure and, if edema is present, an indetation in the pressed area will appear and remain after releasing the pressure. Inflammatory reaction of the local lymph nodes is associated to lymphadenomegaly.

In a particular embodiment, the step of the method of the invention for determining immunoreactivity is carried out by simultaneous inoculation over multiple sites of the body of the subject of the same dose of the immunogenic composition according to the invention and described above. The term "simultaneous inoculation" refers to an inoculation carried out at about the same time in the multiple sites (in a first round). Multiple sites of the body are at least two different sites of the body, preferably at least three, more preferably at least four, at least five, at least six, at least seven, even more preferably at least eight or more. Sites of the body susceptible of being inoculated include, without limitation, cranial dorsal region of the neck, cranial ventral region of the neck, caudal dorsal region of the neck and caudal ventral region of the neck. Further regions susceptible of being inoculated include the caudal skin fold at the root of the tail, which is commonly used for this kind of tests.

In a more particular embodiment, when simultaneous inoculation over multiple sites is performed, one of the sites of the body of the subject where inoculation is performed is the anterior cervical region, corresponding to the area of the neck bounded by the mandible, the anterior border of the sternocleidomastoid muscle, and the anterior midline of the neck. In a more particular embodiment, when simultaneous inoculation over multiple sites is performed, inoculation step (i) is performed in 4 to 8 sites of the body of the subject.

The subject whose reactivity is to be determined is a mammal susceptible of being infected by bacteria of the *Mycobacterium tuberculosis* complex, in particular by *Mycobacterium caprae, Mycobacterium bovis* or *Mycobacterium tuberculosis.* In a more particular embodiment, the subject is a mammal selected from the group consisting of a red deer (*Cervus elaphus*), a wild boar (*Sus scrofa*), a goat (*Capra aegagrus hircus*), a cow *(Bos taurus)* and a sheep (*Ovis aries*).

In a particular embodiment, the bacteria of the *Mycobacterium tuberculosis* complex whose antigens are comprised by the immunogenic composition used in the immunoreactivity method are *Mycobacterium caprae, Mycobacterium bovis* or *Mycobacterium tuberculosis* bacteria, more particularly *Mycobacterium bovis.*

In a particular embodiment, the tuberculosis is bovine tuberculosis or caprine tuberculosis, more particularly bovine tuberculosis.

In a particular embodiment, the bacteria of the *Mycobacterium tuberculosis* complex detected is *M. bovis* or *M. caprae,* preferably *M. bovis.*

Accordingly, the present invention relates to the following aspects:
1. An immunogenic composition comprising antigens from bacteria of the *Mycobacterium tuberculosis* complex, wherein said composition is obtainable by immunopurification from a PPDb by means of an antibody or an antigen-binding fragment thereof, wherein said antibody comprises complementarity-determining regions (CDRs) that comprise amino acid sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.
2. The composition according to aspect 1, wherein the antigen from bacteria of the *Mycobacterium tuberculosis* complex is an antigen selected from the group consisting of MPB70, MPB83, molecular chaperone GroEL, MPB64, 5-methyltetrahydropteroyltriglutamate-homocysteine methyltransferase, ESAT-6-like protein EsxB, elongation factor Tu, ESAT-6, alpha-crystallin and combinations thereof.
3. The composition according to aspect 2, wherein the antigen from bacteria of the *Mycobacterium tuberculosis* complex is selected from the group consisting of MPB70, MPB83 and their combination.
4. The composition according to any of the preceding aspects, wherein the antibody is an IgG antibody.
5. Method for the *in vitro* diagnosis of tuberculosis in a subject comprising
   (i) contacting a sample from said subject with the immunogenic composition according to any one of aspects 1 to 4 and,
   (ii) determining whether complexes between the antigens of the immunogenic composition according to any one of aspects 1 to 4 and antibodies specific for said antigens in the sample are formed,
      wherein the formation of such complexes between the antigens of the immunogenic composition according to any one of aspects 1 to 4 and antibodies specific for said antigens in the sample is indicative of a diagnosis of tuberculosis.
6. The method according to the preceding aspect, wherein the presence of the complexes is determined by an immunoassay selected from the group consisting of ELISA and western blot.
7. Method for the *in vitro* diagnosis of tuberculosis in a subject comprising
   (i) contacting a sample from said subject with the immunogenic composition according to any one of aspects 1 to 4 and,
   (ii) determining the levels of IFN- γ produced as a consequence of step (i), wherein if IFN-γ levels are increased with respect to a reference value, then the subject is diagnosed with tuberculosis.
8. The method according to the preceding aspect, wherein the reference value is determined in a sample from one or more healthy subjects or from one or more subjects not diagnosed with tuberculosis.
9. The method according to any of aspects 5 to 8, wherein the sample is a blood sample.
10. A method for determining the immunoreactivity to a bacteria of the *Mycobacterium tuberculosis* complex in a subject that comprises (i) inoculating intradermically the immunogenic composition according to any of aspects 1 to 4, and
   (ii) determining the skinfold thickness in the inoculation area after the intradermal inoculation,
      wherein if the increase in the skinfold thickness is at least 2 mm greater than a reference value, then the subject is immunoreactive to a bacteria of the *Mycobacterium tuberculosis* complex.
11. The method according to the preceding aspect, wherein local inflammatory clinical signs are further determined, and wherein said signs are selected from the group consisting of oedema, exudation, pain, and inflammatory reaction of the local lymph nodes.
12. The method according to any of aspects 5 to 11, wherein the subject is a mammal susceptible of being infected by a bacteria of the *Mycobacterium tuberculosis* complex, preferably wherein the subject is a mammal selected from the group consisting of a red deer (*Cervus elaphus*), a wild boar (*Sus scrofa*), a goat (*Capra aegagrus hircus*), a cow *(Bos taurus)* and a sheep (*Ovis aries*).
13. The method according to any of aspects 10 to 12, wherein step (i) is carried out by simultaneous inoculation over multiple sites of the body of the subject of the same dose of the immunogenic composition according to any of aspects 1 to 4.
14. The method according to aspect 13, wherein one of the sites of the body of the subject is the anterior cervical region.
15. The method according to any of aspects 10 to 14, wherein step (i) is carried out by inoculation in 4 to 8 sites.
16. Use of the immunogenic composition according to any one of aspects 1 to 4 in the diagnosis of tuberculosis.
17. The use according to aspect 16, wherein the diagnosis is by intradermal inoculation of said immunogenic composition.
18. The method according to any of aspects 5 to 15 or the use according to any of aspects 16 or 17 wherein the tuberculosis is bovine tuberculosis.
19. The immunogenic composition according to any of aspects 1 to 4, the method according to any of aspects 5 to 15 or 18, or the use according to any of aspects 16 to 18 wherein the bacteria of the *Mycobacterium tuberculosis* complex is *Mycobacterium caprae, Mycobacterium bovis* or *Mycobacterium tuberculosis.*
20. An antibody or an antigen-binding fragment thereof, wherein said antibody comprises complementarity-determining regions (CDRs) comprising sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.

The invention is described in detail below by means of the following examples which are to be construed as merely illustrative and not limitative of the scope of the invention.

### EXAMPLES

### Example 1. Diagnostic applications of p22 immunogenic composition

### MATERIALS AND METHODS

### 1. Generation of SIM 18.2 monoclonal antibody

### 1.1. Production of PPDb to be used as an immunogen in the production of monoclonal antibodies (MAb).

The PPDb was obtained according to the standards described in the European Pharmacopoeia, where it is described that the PPDb used in mammals is a preparation of thermally treated products derived from the growth and lysis of *M. bovis.* It is obtained from the water-soluble fractions of *M. bovis* cultures grown in synthetic liquid media subjected to thermal treatment with vapor and subsequent filtering. The active fraction of the filtrate, fundamentally protein components, is isolated by means of a precipitation, washing and resuspension process. This fraction thus obtained was given by the company CZ Veterinaria to be used as an immunogen in the production of MAb.

### 1.2. Immunization and MAb generation

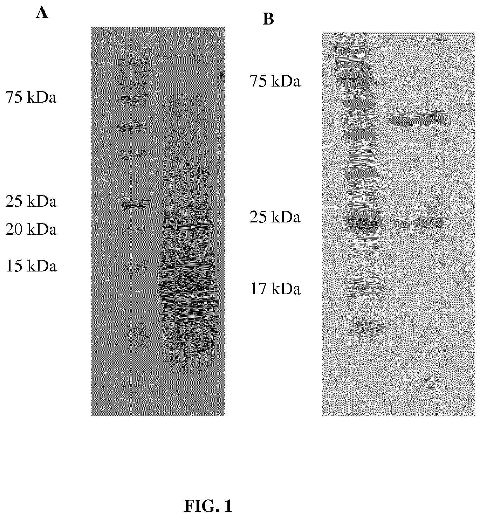

BALB/c mice were intraperitoneally inoculated with 50 µg of PPDb (Figure 1A) emulsified with incomplete Freund's adjuvant. A second dose was administered 15 days later under the same conditions but with 25 µg of PPDb. After one week blood was drawn from the animals, the presence of specific antibodies in the serum was checked and the one having the highest titer was selected as the splenocyte donor. A final booster dose of 25 µg of antigen was administered to this animal 72 hours before sacrificing it. The spleen was extracted on the day of fusion, and the splenocytes were fused with mouse myeloma cells (SP2/0-Ag14). The hybridomas obtained following this method were evaluated as MAb producers by means of capture ELISA, and those recognizing PPDb were selected, confirming their reactivity using western blot (WB) with respect to the same extract.

The selected hybridomas were cloned twice by limiting dilution. One of them, SIM 18.2, was expanded and purified by means of protein G affinity chromatography (HiTrap™ Protein G HP, GE Healthcare) (Figure 1B). The IgG1 kappa isotype of MAb was determined using polyclonal antibodies against different mouse isotypes (goat-anti mouse IgG1, IgG2a, IgG2b or IgG3 HRP, Southern Biotech) and against the two types of light chains (goat-anti mouse kappa/lambda-HRP, Southern Biotech). Likewise, the characterization of the complementarity determining regions (CDRs) was carried out as shown in Example 2.

The specificity of the SIM 18.2 antibody of the invention was determined by means of WB assays using different bovine PPDs and with respect to the PPD of *Mycobacterium avium.*

### 1.3. Identification of p22 product components

The immunogenic product of the invention, referred to hereinafter as p22, originates from the immunopurification of PPDb with SIM 18.2 MAb. The compound recognized by the MAb was purified by means of affinity chromatography using HiTrap NHS-activated HP columns (GE Healthcare) conjugated with the MAb. The purified fraction was subjected to acrylamide/bisacrylamide gel electrophoresis under denaturing and reducing conditions (SDS-PAGE). The bands of the gel recognized by the MAb were identified by means of peptide fingerprinting (ABi 4800 MALDI-TOF/TOF mass spectrometer) (AB SCIEX, Foster City, CA). In parallel, in the eluted fraction containing the immunopurified product without prior treatment, the components thereof were identified by means of Nano-LC (Eksigent 1D Plus) coupled to a triple TOF mass spectrometer (ABSciex 5600 TripleTOF mass spectrometer). The results of peptide identification are shown in Table 1, wherein peptides for which a value of % PSM > 1.5% are shown, and in Table 2, wherein peptides for which a value of % PSM ≤ 1.5% are shown. Information in Table 1: Number: GenInfo Identifier (gi) and Accession.Version sequence identifiers for accession into NCBI database (sequences versions as of 25 November 2015 and corresponding to *Mycobacterium tuberculosis* complex); MW: molecular weight; pI: isoelectric point; Protein score: sum of the ion scores of all peptides that were identified; PSMs: peptide spectrum match, representing the total number of identified peptide spectra matched for the protein; Num. Pept.: different tryptic peptides identified; Coverage: percentage of the protein sequence covered by the identified peptides; %PSM: peptide spectrum match (*%*). Information in Table 2: GenInfo Identifier (gi) and Accession.Version sequence identifiers for accession into NCBI database (sequences versions as of 25 November 2015 and corresponding to *Mycobacterium tuberculosis* complex, unless otherwise indicated).

**Table 1. Identification of peptides of p22 immunogenic composition (% PSM > 1.5%).**

| Number | Description | MW (Da) | pI | Protein score | PSMs | Num. Pept | Coverage | % PSM |
|---|---|---|---|---|---|---|---|---|
| gi\|489509783 WP_003414644.1 | cell surface protein MPB70 | 19153 | 4.75 | 965.97 | 226 | 11 | 29 | 27.59 |
| gi\|489509769 WP_003414630.1 | cell surface protein MPB83 | 22194 | 4.86 | 676.99 | 37 | 9 | 36.4 | 4.52 |
| gi\|489497323 WP_003402236.1 | molecular chaperone GroEL | 56692 | 4.85 | 1335.12 | 24 | 22 | 52.2 | 2.93 |
| gi\|489505073 WP_003409954.1 | Immunogenic protein MPB64 | 25023 | 4.84 | 702.72 | 20 | 11 | 36.4 | 2.44 |
| gi\|489501012 WP_003405914.1 | 5-methyltetrahydropteroyltriglutamate-homocysteine methyltransferase | 81761 | 5.13 | 910.16 | 18 | 17 | 22.8 | 2.20 |
| gi\|489495023 WP_003399940.1 | ESAT-6-like protein EsxB | 10787 | 4.59 | 574.85 | 16 | 10 | 81 | 1.95 |
| gi\|489498552 WP_003403463.1 | elongation factor Tu | 43566 | 5.28 | 935.14 | 16 | 15 | 40.4 | 1.95 |
| gi\|489495046 WP_003399963.1 | ESAT-6 | 9898 | 4.48 | 626.8 | 15 | 9 | 93.7 | 1.83 |
| gi\|489505308 WP_003410189.1 | alpha-crystallin | 16217 | 5 | 682.38 | 13 | 10 | 61.8 | 1.59 |

**Table 2. Identification of peptides of p22 immunogenic composition (% PSM ≤ 1.5%).**

| Number | Description | MW (Da) | pI | Protein score | PSMs | Num. Pept | Coverage | % PSM |
|---|---|---|---|---|---|---|---|---|
| gi\|489504801 WP_003409684.1 | hypothetical protein | 16504 | 4.92 | 454.66 | 10 | 6 | 57.2 | 1.22 |
| gi\|489509793 WP_003414654.1 | protein disulfide oxidoreductase | 18463 | 5.2 | 403.72 | 10 | 9 | 50.3 | 1.22 |
| gi\|489513185 WP_003418028.1 | molecular chaperone GroES *[Mycobacterium]* | 10798 | 4.62 | 469.54 | 10 | 9 | 90 | 1.22 |
| gi\|489516779 WP_003421606.1 | ESAT-6-like protein EsxL | 10012 | 4.72 | 489.93 | 10 | 7 | 50 | 1.22 |
| gi\|489499868 WP_003404775.1 | phosphate-binding protein PstS | 38160 | 5.76 | 347.09 | 9 | 7 | 18.1 | 1.10 |
| gi\|489997754 WP_003900759.1 | diacylglycerol acyltransferase | 35802 | 6.08 | 334.33 | 9 | 7 | 25.1 | 1.10 |
| gi\|554796584 | aconitate hydratase *[Mycobacterium bovis* AN5] | 102661 | 4.95 | 381.93 | 9 | 9 | 10.9 | 1.10 |
| gi\|489505095 WP_003409976.1 | cutinase | 21953 | 5.52 | 300.74 | 8 | 4 | 26.7 | 0.98 |
| gi\|489997833 WP_003900838.1 | heat-shock protein Hsp20 | 17775 | 5.21 | 474.63 | 8 | 7 | 58.5 | 0.98 |
| gi\|489495247 WP_003400164.1 | thiol reductase thioredoxin | 12629 | 5.06 | 377.1 | 7 | 6 | 44.8 | 0.85 |
| gi\|489496900 WP_003401814.1 | molecular chaperone DnaK | 66790 | 4.85 | 266.76 | 7 | 7 | 10.7 | 0.85 |
| gi\|489501664 WP_003406562.1 | hypothetical protein | 24951 | 5.26 | 261.55 | 7 | 5 | 31.1 | 0.85 |
| gi\|489504572 WP_003409456.1 | diacylglycerol acyltransferase | 34697 | 5.62 | 341.11 | 7 | 6 | 22.5 | 0.85 |
| gi\|489506256 WP_003411131.1 | cell wall synthesis protein Wag31 | 28260 | 4.8 | 307.77 | 7 | 6 | 28.5 | 0.85 |
| gi\|489996587 WP_003899599.1 | secretion protein EspC | 10834 | 5.11 | 213.82 | 7 | 4 | 27.2 | 0.85 |
| gi\|499253276 WP_010950816.1 | hypothetical protein | 24557 | 5.35 | 344.03 | 7 | 6 | 48.5 | 0.85 |
| gi\|489496308 WP_003401224.1 | acyl dehydratase | 35950 | 6.49 | 280.05 | 6 | 6 | 25.8 | 0.73 |
| gi\|489500222 WP_003405127.1 | molybdenum cofactor biosynthesis protein | 18476 | 4.53 | 276.7 | 6 | 5 | 35.9 | 0.73 |
| gi\|489501322 WP_003406222.1 | PE family protein | 9610 | 4.56 | 89.91 | 6 | 2 | 31.3 | 0.73 |
| gi\|489503953 WP_003408840.1 | ESAT-6-like protein EsxN | 9982 | 4.76 | 297.89 | 6 | 4 | 50 | 0.73 |
| gi\|489504514 WP_003409398.1 | bacterioferritin | 18421 | 4.5 | 328.59 | 6 | 6 | 43.4 | 0.73 |
| gi\|499253275 WP_010950815.1 | electron transfer flavoprotein subunit alpha | 31700 | 4.71 | 352.03 | 6 | 6 | 31.4 | 0.73 |
| gi\|554797021 | hypothetical protein 0217_06580, partial *[Mycobacterium bovis* AN5] | 16301 | 11.62 | 514.36 | 6 | 5 | 48.5 | 0.73 |
| gi\|489495992 WP_003400908.1 | diacylglycerol acyltransferase | 36794 | 5.92 | 245.73 | 5 | 4 | 12.4 | 0.61 |
| gi\|489496971 WP_003401885.1 | calcium dodecin | 7961 | 8.09 | 189.38 | 5 | 4 | 70.4 | 0.61 |
| gi\|489497426 WP_003402339.1 | heparin-binding hemagglutinin | 21522 | 9.17 | 209.19 | 5 | 5 | 25.6 | 0.61 |
| gi\|489501402 WP_003406301.1 | malate dehydrogenase | 34347 | 4.65 | 205.91 | 5 | 4 | 14 | 0.61 |
| gi\|489501803 WP_003406701.1 | ATP synthase subunit beta | 53153 | 4.86 | 135.21 | 5 | 4 | 9.1 | 0.61 |
| gi\|489504453 WP_003409337.1 | alanine and proline-rich secreted protein Apa | 32667 | 4.93 | 266.17 | 5 | 4 | 18.8 | 0.61 |
| gi\|489506244 WP_003411119.1 | hypothetical protein | 18622 | 5.41 | 179.6 | 5 | 5 | 36.9 | 0.61 |
| gi\|489510308 WP_003415166.1 | ketol-acid reductoisomerase | 36604 | 4.98 | 149.33 | 5 | 4 | 14.8 | 0.61 |
| gi\|489513178 WP_003418021.1 | molecular chaperone GroEL | 55843 | 4.98 | 310.99 | 5 | 5 | 12.8 | 0.61 |
| gi\|489996861 WP_003899869.1 | 3-oxoacyl-ACP reductase | 46893 | 6.04 | 161.47 | 5 | 5 | 13.9 | 0.61 |
| gi\|499252785 WP_010950325.1 | hypothetical protein | 56135 | 4.95 | 178.28 | 5 | 5 | 8.8 | 0.61 |
| gi\|499253317 WP_010950857.1 | adenosylhomocysteinase | 54441 | 5.07 | 216.15 | 5 | 5 | 11.3 | 0.61 |
| gi\|499253342 WP_010950882.1 | dehydrogenase | 30477 | 5.17 | 266.83 | 5 | 5 | 20.7 | 0.61 |
| gi\|499253407 WP_010950947.1 | superoxide dismutase [Fe] | 23008 | 5.96 | 279.41 | 5 | 5 | 38.6 | 0.61 |
| gi\|554794629 | 2,5-diketo-D-gluconic acid reductase *[Mycobacterium bovis* AN5] | 30506 | 4.76 | 280.06 | 5 | 5 | 21.6 | 0.61 |
| gi\|489495617 WP_003400534.1 | single-stranded DNA-binding protein | 17389 | 5.12 | 139.06 | 4 | 3 | 19.5 | 0.49 |
| gi\|489496705 WP_003401621.1 | beta-1,3-glucanase | 32230 | 4.83 | 105.59 | 4 | 3 | 13.6 | 0.49 |
| gi\|489498348 WP_003403259.1 | enoyl-CoA hydratase | 24431 | 5.52 | 168.01 | 4 | 4 | 17.7 | 0.49 |
| gi\|489499444 WP_003404352.1 | TIGR04255 family protein | 30171 | 5.09 | 88.69 | 4 | 3 | 16.2 | 0.49 |
| gi\|489499879 WP_003404786.1 | phosphate-binding protein | 38329 | 5.14 | 126.15 | 4 | 4 | 14.2 | 0.49 |
| gi\|489500440 WP_003405345.1 | peptidase M22 | 10986 | 5.17 | 207.84 | 4 | 4 | 32.7 | 0.49 |
| gi\|489500810 WP_003405712.1 | enoyl-CoA hydratase | 27348 | 4.93 | 148.95 | 4 | 4 | 17.1 | 0.49 |
| gi\|489508727 WP_003413592.1 | universal stress protein | 31724 | 5.46 | 221.73 | 4 | 4 | 15.8 | 0.49 |
| gi\|489508733 WP_003413598.1 | hypoxic response protein 1 | 15646 | 4.96 | 211.85 | 4 | 4 | 35 | 0.49 |
| gi\|489516087 WP_003420920.1 | ferritin BfrB | 20429 | 4.73 | 227.62 | 4 | 4 | 29.3 | 0.49 |
| gi\|489998280 WP_003901285.1 | catalase peroxidase | 80649 | 5.05 | 184.16 | 4 | 4 | 6.6 | 0.49 |
| gi\|489998779 WP_003901784.1 | oxidoreductase | 49455 | 8.32 | 197.28 | 4 | 4 | 9 | 0.49 |
| gi\|489496532 WP_003401448.1 | S-adenosylmethionine-dependent methyltransferase | 33070 | 4.88 | 109.24 | 3 | 3 | 11.3 | 0.37 |
| gi\|489496942 WP_003401856.1 | class II fructose-bisphosphate aldolase | 36705 | 5.49 | 95.76 | 3 | 3 | 9 | 0.37 |
| gi\|489497388 WP_003402301.1 | dihydrolipoyl dehydrogenase | 49392 | 5.53 | 135.36 | 3 | 3 | 7.8 | 0.37 |
| gi\|489499877 WP_003404784.1 | phosphate-binding protein | 38054 | 4.97 | 147.21 | 3 | 3 | 9.2 | 0.37 |
| gi\|489502497 WP_003407390.1 | glyceraldehyde-3-phosphate dehydrogenase | 36072 | 5.19 | 124.7 | 3 | 3 | 9.1 | 0.37 |
| gi\|489503153 WP_003408045.1 | transcriptional regulator | 22655 | 5.02 | 140.59 | 3 | 3 | 20 | 0.37 |
| gi\|489503193 WP_003408085.1 | universal stress protein | 15303 | 5.51 | 69.72 | 3 | 3 | 21.2 | 0.37 |
| gi\|489503632 WP_003408522.1 | hypothetical protein | 10646 | 6.18 | 85.27 | 3 | 3 | 37.2 | 0.37 |
| gi\|489506576 WP_003411450.1 | dihydrolipoyllysine-residue acetyltransferase component of pyruvate dehydrogenase complex | 57099 | 4.9 | 137.06 | 3 | 3 | 5.8 | 0.37 |
| gi\|489506601 WP_003411475.1 | glutamine synthetase 1 | 53674 | 5.04 | 142.34 | 3 | 3 | 6.7 | 0.37 |
| gi\|489507723 WP_003412592.1 | nucleoside-diphosphate kinase | 14499 | 5.34 | 126.16 | 3 | 3 | 27.2 | 0.37 |
| gi\|489508128 WP_003412996.1 | X-Pro dipeptidase | 38813 | 5.26 | 96.79 | 3 | 3 | 8.3 | 0.37 |
| gi\|489509636 WP_003414497.1 | enoyl-CoA hydratase | 26752 | 5.82 | 145.04 | 3 | 3 | 17.7 | 0.37 |
| gi\|489513510 WP_003418351.1 | DNA-directed RNA polymerase subunit alpha | 37729 | 4.64 | 110.6 | 3 | 2 | 6.3 | 0.37 |
| gi\|489514552 WP_003419389.1 | uncharacterized protein | 18960 | 4.93 | 127.86 | 3 | 2 | 16.5 | 0.37 |
| gi\|489515950 WP_003420783.1 | MPT51/MPB51 antigen | 31069 | 6.13 | 232.24 | 3 | 3 | 12 | 0.37 |
| gi\|489996815 WP_003899824.1 | maltokinase | 49996 | 5.06 | 121.95 | 3 | 3 | 6.2 | 0.37 |
| gi\|489997328 WP_003900333.1 | S-adenosylmethionine synthase | 43112 | 4.95 | 127.11 | 3 | 3 | 9.2 | 0.37 |
| gi\|489997788 WP_003900793.1 | hypothetical protein | 31904 | 6.97 | 113.58 | 3 | 3 | 13.2 | 0.37 |
| gi\|490008984 WP_003911788.1 | pyruvate dehydrogenase E1 component | 103560 | 5.62 | 72.32 | 3 | 3 | 4.3 | 0.37 |
| gi\|499252920 WP_010950460.1 | 3-hydroxyacyl-CoA dehydrogenase | 76178 | 5.42 | 98.11 | 3 | 3 | 5 | 0.37 |
| gi\|499253107 WP_010950647.1 | NAD(P)H nitroreductase | 36736 | 5.28 | 124.06 | 3 | 3 | 11.8 | 0.37 |
| gi\|499253254 WP_010950794.1 | elongation factor Ts | 28859 | 5.26 | 142.84 | 3 | 3 | 12.5 | 0.37 |
| gi\|489496413 WP_003401329.1 | uncharacterized protein | 10935 | 4.47 | 68.03 | 2 | 2 | 15.5 | 0.24 |
| gi\|489498141 WP_003403052.1 | hypothetical protein | 23714 | 4.57 | 105.99 | 2 | 2 | 8.3 | 0.24 |
| gi\|489499504 WP_003404412.1 | hypothetical protein | 16335 | 5.1 | 137.76 | 2 | 2 | 19 | 0.24 |
| gi\|489499758 WP_003404665.1 | type II citrate synthase | 48010 | 5.36 | 104.24 | 2 | 2 | 5.6 | 0.24 |
| gi\|489502071 WP_003406967.1 | uncharacterized protein | 12933 | 5.54 | 80.08 | 2 | 1 | 11.4 | 0.24 |
| gi\|489502422 WP_003407315.1 | hypothetical protein | 24625 | 7.77 | 68.92 | 2 | 2 | 7.6 | 0.24 |
| gi\|489503098 WP_003407990.1 | indole-3-glycerol-phosphate synthase | 28098 | 5.07 | 107.7 | 2 | 2 | 7.4 | 0.24 |
| gi\|489503959 WP_003408846.1 | ESX secretion-associated protein EspG | 32471 | 4.86 | 62.41 | 2 | 2 | 8.3 | 0.24 |
| gi\|489505156 WP_003410037.1 | hypothetical protein | 27398 | 4.74 | 64.82 | 2 | 2 | 8.1 | 0.24 |
| gi\|489506350 WP_003411225.1 | division/cell wall cluster transcriptional repressor MraZ | 15948 | 6.06 | 76.96 | 2 | 2 | 18.9 | 0.24 |
| gi\|489506480 WP_003411354.1 | hypothetical protein | 16314 | 4.75 | 63.44 | 2 | 2 | 20.1 | 0.24 |
| gi\|489506814 WP_003411688.1 | hypothetical protein | 31964 | 5.07 | 112.83 | 2 | 2 | 11 | 0.24 |
| gi\|489507839 WP_003412708.1 | energy-dependent translational throttle protein EttA | 62039 | 5.17 | 65.29 | 2 | 2 | 3 | 0.24 |
| gi\|489508089 WP_003412957.1 | hypothetical protein | 25400 | 9.52 | 73.55 | 2 | 2 | 7.1 | 0.24 |
| gi\|489510012 WP_003414872.1 | hypothetical protein | 24263 | 5.03 | 98.93 | 2 | 2 | 11.2 | 0.24 |
| gi\|489511113 WP_003415965.1 | NADP-dependent alcohol dehydrogenase C | 37420 | 5.11 | 98.81 | 2 | 2 | 6.4 | 0.24 |
| gi\|489511259 WP_003416111.1 | hypothetical protein | 30581 | 5.18 | 68.14 | 2 | 2 | 9.2 | 0.24 |
| gi\|489512307 WP_003417156.1 | acetyl-/propionyl-CoA carboxylase subunit alpha | 63928 | 5.48 | 70.24 | 2 | 2 | 5.3 | 0.24 |
| gi\|489514714 WP_003419551.1 | ESX-1 secretion-associated protein EspA | 39922 | 5.19 | 81.65 | 2 | 2 | 9.7 | 0.24 |
| gi\|489514891 WP_003419728.1 | cAMP receptor protein | 24776 | 9.57 | 53.11 | 2 | 2 | 8.5 | 0.24 |
| gi\|489515575 WP_003420410.1 | hypothetical protein | 24913 | 9.3 | 63.32 | 2 | 2 | 7.9 | 0.24 |
| gi\|489996011 WP_003899041.1 | glycogen accumulation regulator GarA | 17240 | 4.29 | 88.56 | 2 | 2 | 12.3 | 0.24 |
| gi\|489996047 WP_003899077.1 | hypothetical protein | 20377 | 6.02 | 83.45 | 2 | 2 | 16.4 | 0.24 |
| gi\|489996728 WP_003899737.1 | ESX-1 secretion-associated protein EspF | 10611 | 6.02 | 67.81 | 2 | 2 | 22.3 | 0.24 |
| gi\|490005593 WP_003908487.1 | hypothetical protein | 36618 | 5.26 | 49.3 | 2 | 1 | 2.3 | 0.24 |
| gi\|490009413 WP_003912209.1 | PPOX class F420-dependent oxidoreductase | 14732 | 5.2 | 126.07 | 2 | 2 | 19.9 | 0.24 |
| gi\|490015326 WP_003917895.1 | cyclase | 16050 | 4.55 | 73.35 | 2 | 2 | 11.6 | 0.24 |
| gi\|490015893 WP_003918447.1 | ATP-dependent Clp protease proteolytic subunit | 21125 | 4.88 | 118.9 | 2 | 2 | 11.3 | 0.24 |
| gi\|499252886 WP_010950426.1 | 3-hydroxyacyl-ACP dehydratase | 14895 | 6.08 | 81.73 | 2 | 2 | 14.1 | 0.24 |
| gi\|499253075 WP_010950615.1 | hypothetical protein | 69700 | 4.7 | 90.06 | 2 | 2 | 3.1 | 0.24 |
| gi\|554793963 | hypothetical protein 0217_19000 *[Mycobacterium bovis* AN5] | 28252 | 5.84 | 158.68 | 2 | 2 | 13.2 | 0.24 |
| gi\|554794450 | hypothetical protein 0217_17035 *[Mycobacterium bovis* AN5] | 9884 | 4.86 | 72.31 | 2 | 2 | 25.3 | 0.24 |
| gi\|554797149 | hypothetical protein 0217_06570, partial *[Mycobacterium bovis* AN5] | 22804 | 3.8 | 129.53 | 2 | 2 | 10.3 | 0.24 |
| gi\|554799228 | peptidyl-prolyl cis-trans isomerase *[Mycobacterium bovis* AN5] | 19274 | 5.59 | 83.52 | 2 | 2 | 14.8 | 0.24 |

### 2. Diagnostic applications of p22

The product that has been obtained and characterized was used in diagnostic tests aimed at identifying animals infected by complex *M. tuberculosis* species.

### 2.1. Determination of specific antibodies

Those animals with specific antibodies for the *M. tuberculosis* complex were identified by means of indirect ELISA. According to the indirect ELISA protocol developed, the ELISA plates were covered overnight at 4°C or for 2 hours at 37°C with 50 µl with a 10 µg/ml preparation of p22. After this incubation period, the plates were blocked with 100 µl of 5% milk in PBS for 30 minutes at 37°C. After washing with 0.05*%* PBS/Tween (PBS-T), 50 µl of target serum were added in duplicate to a 1/100 dilution in 5% milk and the plates were incubated for 1 hour at 37°C. After this time the plates were washed 3 times with PBS-T and the secondary antibody was added, *ad hoc* for each species (goat, wild boar or deer) labeled with peroxidase, incubating for 30 minutes at room temperature. The reactivity of the sera was clearly seen using OPD (O-phenyldiamine) as a chromophore at a concentration of 1 mg/ml in citrate-phosphate buffer (0.05 and 0.1 M respectively), pH 4.5. The development of the colorimetric reaction was stopped with 3 N H₂SO₄, and optical density was measured in a plate spectrophotometer at a wavelength of 492 nm.

### 2.2. Cell reactivity

To study the cell immune response, a population of Guadarrama goats (n=60) more than 2 years of age and naturally infected with tuberculosis was selected. Natural infection by *Mycobacterium caprae* was confirmed in the herd, and the animals tested positive in the official diagnostic test (single intradermal reaction) two months before the present study.

To assess the response obtained using the purified protein fractions, *in vivo* (IDTB test) and *in vitro* (IFN-γ detection test) diagnostic techniques were used.

### • Intradermal test (IDTB)

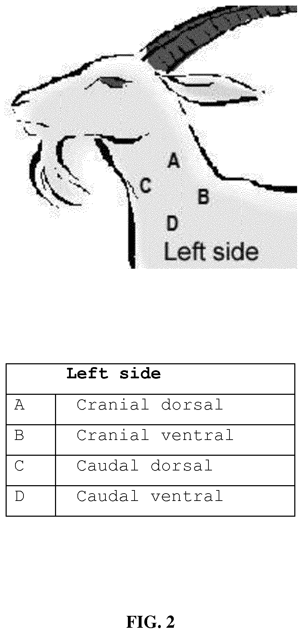

To conduct the IDTB, four inocula were administered intradermally per animal following the model described in detail in Figure 2. The antigens used and the position in which each of them was inoculated were:
1. 0.6 mg/ml of p22--Cranial dorsal.
2. Commercial PPDb--Cranial ventral.
3. Commercial PPDa--Caudal dorsal.
4. PBS-Caudal ventral

All the antigens were inoculated in a volume of 0.1 ml using in all cases insulin syringes (0.5 x 16 mm). The results were interpreted according to the strict criteria listed in Directive 64/432.

### • IFN-γ detection test

For the *in vitro* assessment of the biological activity of the proteins, whole blood from those animals which had been used for conducting the IDTB was used. The blood was drawn before inoculation of the different antigens and it was stimulated with the same preparations used in the IDTB with the methodological modifications required for this type of test:
1. 30 µg/100 µl of p22.
2. PPDb.
3. PPDa (PPD from *Mycobacterium avium*).
4. PBS.
5. Mitogen.

Briefly, a blood sample from one and the same animal was used to prepare several aliquots to which 100 µl of each of the mentioned antigens were added; said aliquots were incubated from 18 to 24 hours at 37°C and the samples were subsequently centrifuged at 770 x g for 15 minutes. The plasma obtained was used in Bovigam commercial ELISA (Thermo Scientific) for specific IFN-γ detection following the manufacturer's instructions.

The results were interpreted as described in the National Bovine Tuberculosis Eradication Program, considering that an animal is positive when the following is met:
- Full PPDb OD -PBS OD ≥ 0.05 and
- PPDb OD > PPDa OD.

When p22 was used as an immunogen, an animal was considered positive when:
- Stimulated sample OD -PBS OD ≥ 0.05.

### RESULTS

### 1. SIM 18.2 MAb

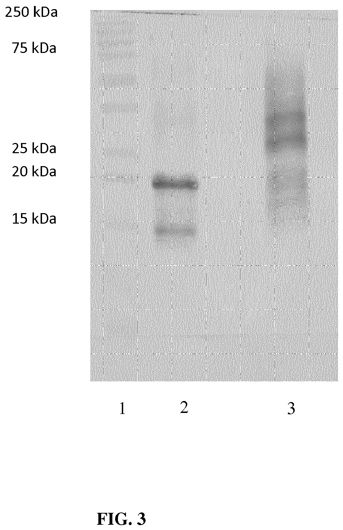

The results obtained demonstrated that the SIM 18.2 MAb specifically and quantitatively recognizes a protein of about 22 and 15 kDa, under reducing conditions, present in PPDb but not in PPDa. The intensity of the band in the WB was proportional to the intensity of the protein stained with Coomassie in an SDS-PAGE gel of the same sample. However, under non-reducing conditions the antibody recognizes bands of between 30 and 40 kDa (Figure 3).

### 2. Identification of p22 product components

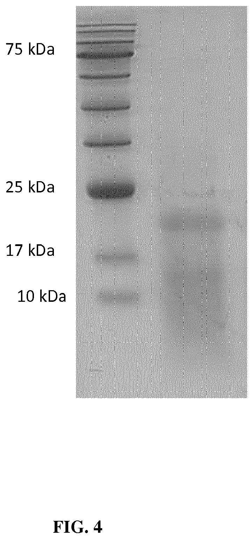

After immunopurification with the SIM 18.2 MAb from PPDb, two bands with a molecular weight of about 20 and 15 kDa were observed in the gel (Figure 4). After processing and mass spectrometry analysis of the components located in the 20 kDa band, the presence of MPB70 and MPB83 proteins was confirmed with a score of 283 and 211, respectively. Since both proteins are homologous (76% identity), the explanation is that the MAb recognizes the same epitope in both proteins. As regards the 15 kDa band, the two MPB70 and MPB83 proteins were likewise identified with a score of 210 and 178, which is compatible with a natural hydrolysis process described in the literature for these proteins (Wiker HG 2009 Scand. J. Immunol. 69: 492-499).

The reactivity of the MAb being different under reducing and non-reducing conditions is the result of the MPB70 protein and the MPB83 protein having cysteine residues in their sequence providing them the capacity to form interchain disulfide bridges giving rise to 30, 35 and 40 kDa dimers.

Analysis by means of triple TOF indicates that the p22 product is a multiprotein compound formed by a total of 116 proteins and not only by MPB70/83 proteins. Although MPB70/83 are the main proteins in both determinations, other proteins different from MPB70/83, about 20 or so, are in the three analyses conducted above 10 according to the Peptide Spectrum Match (PSM), indicating a quantitatively significant proportion in the p22 composition.

### 3. Diagnostic applications of p22: in vivo bovine tuberculosis diagnostic methods

### Humoral immune response

### Experiments carried out in deer and wild boars:

The serum from deer and wild boars with lesions compatible with tuberculosis (TB) and tested positive in culture isolation and PCR identification for *M. bovis* from (TB positive) tissues, and the serum from deer of a TB free (TB negative) farm were analyzed by means of ELISA to determine the sensitivity, specificity and positive predictive value (PPV) and negative predictive value (NPV) of this technique using PPDb, MPB70 recombinant protein and the compound object of the patent (p22) as antigens for the diagnosis of TB and comparing them.

After the ELISA study of 140 deer sera, the results obtained determined that by using p22 as an antigen for detecting anti-*M*. *bovis* antibodies, sensitivity and specificity of the technique increase by about 10%; the predictive values also increase (Tables 2A, B and C).

**Table 2: Evaluation and comparison of the ELISA performed with p22 (A) with respect to PPDb (B) and MPB70 (C) for the diagnosis of TB in deer. PPV: positive predictive value; NPV: negative predictive value**

| (A) | | *TB positive | †TB negative | Total |
|---|---|---|---|---|
| | Positive | 20 | 1 | 21 |
| ELISA p22 | Negative | 12 | 107 | 119 |
| | Total | 32 | 108 | 140 |
| | | | | |

| | Sensitivity | Specificity | PPV | NPV |
|---|---|---|---|---|
| | 62.5% | 99% | 95.2% | 89.9% |
| | | | | |

| (B) | | *TB positive | †TB negative | Total |
|---|---|---|---|---|
| | Positive | 23 | 11 | 34 |
| ELISA PPDb | Negative | 9 | 97 | 106 |
| | Total | 32 | 108 | 140 |
| | | | | |

| | Sensitivity | Specificity | PPV | NPV |
|---|---|---|---|---|
| | 71.8% | 89.8% | 67.6% | 91.5% |

| (C) | | *TB positive | †TB negative | Total |
|---|---|---|---|---|
| | Positive | 19 | 5 | 24 |
| ELISA MPB70 | Negative | 13 | 103 | 116 |
| | Total | 32 | 108 | 140 |
| | | | | |

| | Sensitivity | Specificity | PPV | NPV |
|---|---|---|---|---|
| | 59,3% | 95,3% | 79,1% | 88,8% |

| | | | | |
|---|---|---|---|---|
| * Determined by means of culture of *M .bovis* †Animals from a TB-free farm | | | | |

Similar results were observed in wild boar sera, in which sensitivity and specificity increased by 5 and 7%, respectively, using p22 as an antigen for detecting anti-*M*. *bovis* antibodies by indirect ELISA after the analysis of 69 sera from wild boars positive for tuberculosis and another 69 sera from wild boars negative for this disease (Tables 3A and B).

**Table 3: Evaluation and comparison of the ELISA performed with p22 (A) and with PPDb (B) for the diagnosis of TB in wild boars. PPV: positive predictive value; NPV: negative predictive value**

| (A) | | *TB positive | †TB negative | Total |
|---|---|---|---|---|
| | Positive | 53 | 4 | 57 |
| ELISA p22 | Negative | 16 | 65 | 81 |
| | Total | 69 | 69 | 138 |
| | | | | |

| | Sensitivity | Specificity | PPV | NPV |
|---|---|---|---|---|
| | 76.8% | 94.2% | 92.9% | 80.2% |
| | | | | |

| (B) | | *TB positive | †TB negative | Total |
|---|---|---|---|---|
| ELISA PPDb | Positive | 48 | 6 | 54 |
| | Negative | 21 | 63 | 84 |
| | Total | 69 | 69 | 138 |
| | Sensitivity | Specificity | PPV | NPV |
| | 69.5% | 91.3% | 88.8% | 75% |

### Experiments carried out in goats:

For this study, the serum of 4 goats from a herd confirmed as *M. caprae* positive and positive in the official diagnosis test (single IDTB) two months before the present study was used. The specific antibodies for different *M. bovis* antigens were determined by means of indirect ELISA, comparing p22 object of the patent, PPDb, p10 and p15 proteins (ESAT-6 and HSPX, respectively, present in the p22 antigenic preparation), MPB70 and MPB83 recombinant proteins, as well as a mixture of both.

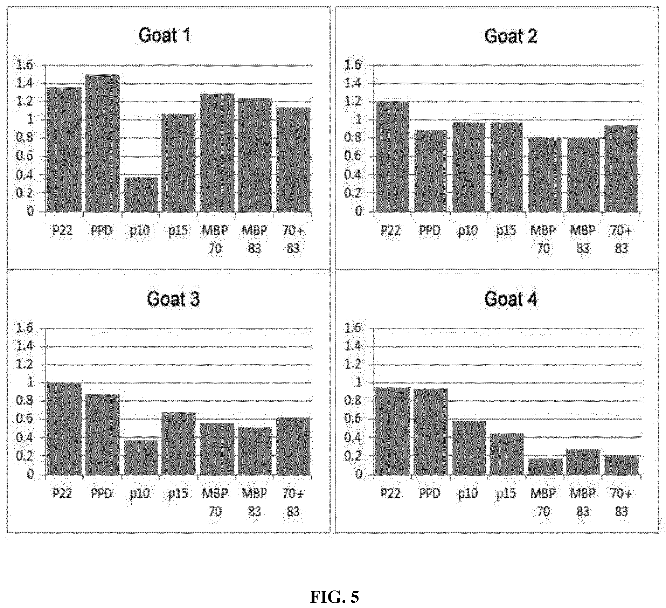

The results showed that in animals with specific anti-MPB70/83 protein antibodies the titer is the same regardless of whether the recombinant proteins or p22 complex (goat 1 and 2) are used. However, there are animals that do not have specific antibodies against the recombinant proteins, or they have a very low titer, whereas they do react with the p22 complex. This is due to the presence of antibodies against other proteins of the p22 complex other than MPB70/83, such as p10 and p15. In these cases, the animals would be erroneously diagnosed as TB negative when recombinant proteins are used as an antigen, whereas they would be correctly diagnosed as positive animals (goats 3 and 4) when the p22 complex is used (Table 4 and Figure 5).

**Table 4: Optical densities of the serum of 4 goats with respect to different M. bovis antigens. 70+83: MPB70 + MPB83**

| | p22 | PPDb | p10 | p15 | MBP 70 | MBP 83 | MPB70 + MPB83 |
|---|---|---|---|---|---|---|---|
| Goat 1 | 1.35 | 1.49 | 0.37 | 1.06 | 1.29 | 1.24 | 1.13 |
| Goat 2 | 1.2 | 0.89 | 0.97 | 0.97 | 0.81 | 0.79 | 0.94 |
| Goat 3 | 1 | 0.88 | 0.37 | 0.68 | 0.56 | 0.52 | 0.62 |
| Goat 4 | 0.95 | 0.93 | 0.59 | 0.44 | 0.18 | 0.27 | 0.21 |
| Control - | 0.07 | 0.06 | 0.06 | 0.12 | 0.08 | 0.07 | 0.07 |

### Cell immune response

Experiments carried out in goats:

### • Intradermal test (IDTB)

For this study, animals testing positive to the IDTB technique in a positive herd (n=60) and in a negative herd vaccinated against paratuberculosis (n=60) were analyzed.

In the case of the positive herd, the results by means of intradermal p22 inoculation are similar to those obtained by means of PPDb inoculation, 32 animals testing positive to said test (53.33*%*) being detected with PPDb, whereas 33 animals reacting to said test (55%) were detected with 0.6 mg/ml of p22. Furthermore, two animals having an increase in the skin fold less than 2 mm were considered positive with PPDb and one animal with p22 based on the detection of local clinical signs at specific point of inoculation.

However, in the farm testing negative the number of false positives drops from 21 animals when inoculated with PPDb to 13 when using p22 as an antigen (Table 5). In view of the data observed, the p22 antigenic preparation is a more specific reagent for the diagnosis of bovine tuberculosis.

**Table 5: Comparison of the IDTB with PPDb and p22 for the diagnosis of TB in goats.**

| | PPDb (1 mg/mL) | p22 (0.6 mg/mL) |
|---|---|---|
| Farm testing positive (n=60) | 32 | 33 |
| Farm testing negative (n=60) | 21 | 13 |

### • IFN-γ detection test

In the *in vitro* test all the OD values obtained with the mitogen were greater than 0.05, showing correct lymphocyte viability and functionality as contemplated in the manufacturer's indications.

The number of animals reacting to the IFN-γ test using 0.3 mg/ml of p22 and PPDb was greater compared with the intradermal reaction. The number of animals considered positive were 47 (78.33*%*) and 53 (88.33*%*), respectively.

### Example 2. CDR characterization of SIM 18.2 monoclonal antibody

### MATERIALS AND METHODS

### 1.1. Hybridomas culture and monoclonal antibodies isotyping

Anti-p22 hybridoma cells from clone PA377-18.2.1 were grown as monolayers at 37°C and 5% CO2 in RPMI-1640 medium with L-glutamine and sodium bicarbonate (R0883, Sigma Aldrich), supplemented with 10% fetal bovine serum (F7524, Sigma Aldrich), 50 U/ml penicillin and 50 µg/ml streptomycin (P4458, Sigma Aldrich). Hybridoma cells were collected for mRNA extraction and the isotype of each secreted monoclonal antibody (MAb) was determined from supernatants using the IsoQuick™ Kit for Mouse Monoclonal Isotyping (ISOQ5, Sigma Aldrich). The MAb isotype secreted by hybridoma was IgG1κ.

### 1.2. Preparation of mRNA

The total RNA of the hybridoma was extracted from 3x10⁷ cells using TRIZOL Reagent (15596-026, Life Technologies) and chloroform (1.02445.1000, Merck), followed by centrifugation to separate the aqueous phase. The mRNA was then isolated by precipitation with 70% ethanol (1.00983.1000, Merck) and purified with RNeasy Mini Kit (74104, QIAGEN) following the manufacturer instructions.

Concentration and purity of purified mRNA (PA377-18.2.1) was assessed with Nanodrop ND 2000 spectrophotometer (Thermo Scientific), showing valid 260/280 and 260/230 ratios, as follows:
- RNA concentration: 320.7 ng/µl
- Ratio 260/280: 1.97
- Ratio 260/230: 1.98

### 1.3. First strand cDNA synthesis

About 5 µg mRNA obtained from the hybridoma was added to a PCR tube containing 1 µl oligo(dT)₂₀ primers (18418-020, Life Technologies), 1 µl dNTPs (R0192, Thermo Scientific) and adding ultrapure water to reach an intermediate reaction volume of 13 µl. After 5 min denaturation at 65°C, 4 µl of 5X first-strand buffer, 1 µl 0.1M DTT, 1 µl RNaseOUT Ribonuclease Inhibitor (10777-019, Life Technologies) and 1 µl SuperScript™ III Reverse Transcriptase (18080-093, Life Technologies) were added to each tube for reverse transcription, reaching a final reaction volume of 20 µl, followed by 1 cycle of 5 min at 25°C, 60 min at 50°C and 15 min at 70°C. Finally, 1 µl Ribonuclease H (18021-014, Life Technologies) was added to the tube and the reaction was incubated 20 min at 37°C. One microliter of each reaction was analyzed by agarose gel electrophoresis.

### 1.4. PCR amplification of V_{L} and V_{H}

Several commercial Taq DNA polymerases (Life Technologies) were successfully used for amplifications of V_{H} and V_{L}. For amplification of V_{L} from hybridoma either λ or κ primers were chosen according to the isotype. PCR reactions were performed in 20 µl volumes, containing 1 µl of cDNA reaction, 0.8 µl of VL_Back and 0.8 µl of VL_For primer mixes for amplification of V_{L} or 0.8 µl of VH_Back and 0.8 µl of VH_For primer mixes for amplification of V_{H}, 2 µl dNTPs (2mM), 0.5 µl of MgCl₂ (25mM), 4 µl betaine and 2 µl reaction buffer 10X supplied by the manufacturers (primer sequences as described in Krebber A et al. 1997 J Immunol Methods 201: 35-55).

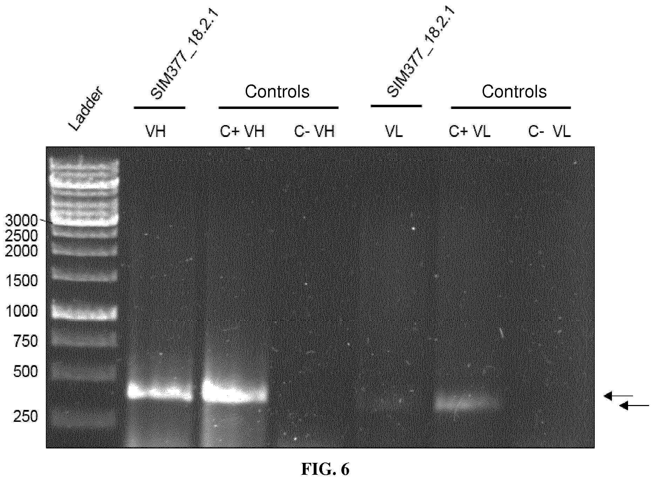

After 3 min denaturation at 94°C, 0.15 µl of KOD (2.5 units/µl) DNA polymerase (71085-3, Novagen) were added, followed by 32 cycles of 30 sec at 94°C, 30 sec at 50°C, 1 min at 72°C, and 1 cycle of 7 min at 72°C. One microliters of each PCR reaction was analyzed by agarose gel electrophoresis (Figure 6).

### 1.5. Cloning of PCR fragments into pGEM®-T Easy Vector

The full length PCR products of V_{L} and V_{H} were purified by preparative agarose gel electrophoresis in combination with the GeneJET™ Gel Extraction Kit (K0691, Thermo Scientific), following manufacturer's instructions.

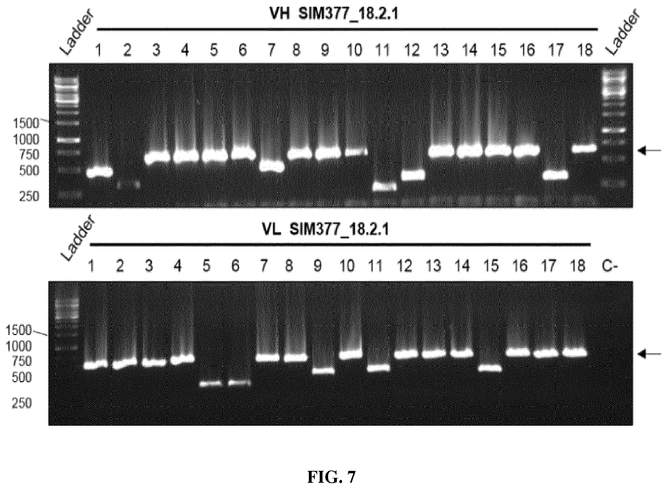

The gel-purified V_{L} and V_{H} fragments were separately cloned into pGEM®-T Easy Vector System II (A1380, Promega) following manufacturer's instructions and transformed into E. coli JM109™ Competent Cells (A1380, Promega). Colony analysis was performed by PCR reaction (Figure 7).

### 1.6. Sequencing and CDRs identification

PCR positive colonies were cultured and grown in Luria Broth media (1551, Pronadisa) containing ampicillin (A9518, Sigma Aldrich) followed by plasmidic DNA extraction using GeneJET™ Plasmid Miniprep Kit (K0503, Thermo Scientific) according to manufacturer's instructions. The nucleic acid sequences (25 V_{L} clones and 12 V_{H} clones) were determined by sequencing using the ABI Prism Big Dye™ Terminator system and the ABI 3730 multicapillary DNA analyzer (Applied Biosystems). Geospiza's FinchTV software was used to view and analyze DNA sequences on Windows.

The complementarity (or specificity)-determining regions (CDRs) from the mouse-derived hybridoma was identified for clone SIM377_18.2.1 following the set of rules of Kabat and Chothia numbering schemes (Table 6).

**Table 6. Hybridomas CDRs identification based on Kabat and Chothia numbering schemes**

| VL - CDRs hybridoma PA377-18.2.1 | | |
|---|---|---|
| CDR-L1 | CDR-L2 | CDR-L3 |
| RSSQSIVHSNGNTYLE | KVSNRFS | FQGSHVPWT |

| VH - CDRs hybridoma PA377-18.2.1 | | |
|---|---|---|
| CDR-H1 | CDR-H2 | CDR-H3 |
| GYSFTGYFMN | RINPYNGDTFYNQKFKG | WDGAPTYFFDY |

## Claims

1. An immunogenic composition comprising antigens from bacteria of the *Mycobacterium tuberculosis* complex, wherein said composition is obtainable by immunopurification from a bovine tuberculin purified protein derivative (PPDb) by means of an antibody or an antigen-binding fragment thereof, wherein said antibody comprises complementarity-determining regions (CDRs) that comprise amino acid sequences RSSQSIVHSNGNTYLE (CDR-L1) (SEQ NO: 1), KVSNRFS (CDR-L2) (SEQ NO: 2), FQGSHVPWT (CDR-L3) (SEQ NO: 3), GYSFTGYFMN (CDR-H1) (SEQ NO: 4), RINPYNGDTFYNQKFKG (CDR-H2) (SEQ NO: 5) and WDGAPTYFFDY (CDR-H3) (SEQ NO: 6) or variants thereof.

2. The composition according to claim 1, wherein the antigen from bacteria of the *Mycobacterium tuberculosis* complex is an antigen selected from the group consisting of MPB70, MPB83, molecular chaperone GroEL, MPB64, 5-methyltetrahydropteroyltriglutamate-homocysteine methyltransferase, ESAT-6-like protein EsxB, elongation factor Tu, ESAT-6, alpha-crystallin and combinations thereof, preferably selected from the group consisting of MPB70, MPB83 and their combination.

3. Method for the *in vitro* diagnosis of tuberculosis in a subject comprising
(i) contacting a sample, preferably a blood sample, from said subject with the immunogenic composition according to any one of claims 1 or 2 and,
(ii) determining whether complexes between the antigens of the immunogenic composition according to any one of claims 1 or 2 and antibodies specific for said antigens in the sample are formed,
wherein the formation of such complexes between the antigens of the immunogenic composition according to any one of claims 1 or 2 and antibodies specific for said antigens in the sample is indicative of a diagnosis of tuberculosis.

4. The method according to the preceding claim, wherein the presence of the complexes is determined by an immunoassay selected from the group consisting of enzyme-linked immunosorbent assay (ELISA) and western blot.

5. Method for the *in vitro* diagnosis of tuberculosis in a subject comprising
(i) contacting a sample from said subject with the immunogenic composition according to any one of claims 1 to 4 and,
(ii) determining the levels of interferon-γ (IFN- y) produced as a consequence of step (i),
wherein if IFN-γ levels are increased with respect to a reference value, then the subject is diagnosed with tuberculosis.

6. The method according to the preceding claim, wherein the reference value is determined in a sample from one or more healthy subjects or from one or more subjects not diagnosed with tuberculosis.

7. Method for determining the immunoreactivity to bacteria of the *Mycobacterium tuberculosis* complex in a subject that comprises
(i) inoculating intradermically the immunogenic composition according to any of claims 1 or 2, and
(ii) determining the skinfold thickness in the inoculation area after the intradermal inoculation,
wherein if the increase in the skinfold thickness is at least 2 mm greater than a reference value, then the subject is immunoreactive to a bacteria of the *Mycobacterium tuberculosis* complex.

8. The method according to the preceding claim, wherein local inflammatory clinical signs selected from the group consisting of oedema, exudation, pain, and inflammatory reaction of the local lymph nodes are further determined.

9. The method according to any of claims 3 to 8, wherein the subject is a mammal susceptible of being infected by a bacteria of the *Mycobacterium tuberculosis* complex, preferably wherein the subject is a mammal selected from the group consisting of a red deer *(Cervus elaphus),* a wild boar *(Sus scrofa*)*,* a goat (*Capra aegagrus hircus),* a cow *(Bos taurus)* and a sheep (*Ovis aries*).

10. The method according to any of claims 7 to 9, wherein step (i) is carried out by simultaneous inoculation over multiple sites of the body of the subject of the same dose of the immunogenic composition according to any of claims 1 or 2.

11. Use of the immunogenic composition according to any one of claims 1 or 2 in the diagnosis of tuberculosis.

12. The use according to the preceding claim, wherein the diagnosis is by intradermal inoculation of said immunogenic composition.

13. The method according to any of claims 3 to 10 or the use according to any of claims 11 or 12 wherein the tuberculosis is bovine tuberculosis.

14. The immunogenic composition according to any of claims 1 or 2, the method according to any of claims 3 to 10 or 13, or the use according to any of claims 11 to 13 wherein the bacteria of the *Mycobacterium tuberculosis* complex is *Mycobacterium caprae, Mycobacterium bovis* or *Mycobacterium tuberculosis.*

15. An antibody or an antigen-binding fragment thereof, wherein said antibody comprises complementarity-determining regions (CDRs) comprising sequences RSSQSIVHSNGNTYLE (CDR-L1) (SEQ ID NO: 1), KVSNRFS (CDR-L2) (SEQ ID NO: 2), FQGSHVPWT (CDR-L3) (SEQ ID NO: 3), GYSFTGYFMN (CDR-H1) (SEQ ID NO: 4), RINPYNGDTFYNQKFKG (CDR-H2) (SEQ ID NO: 5) and WDGAPTYFFDY (CDR-H3) (SEQ ID NO: 6) or variants thereof.


SEQUENCE LISTING

<110>  CZ Veterinaria S.A.

<120>  METHODS AND COMPOSITIONS FOR TUBERCULOSIS DIAGNOSIS

<130>  P12219EP00

<160>  6

<170>  PatentIn version 3.5

<210>  1
<211>  16
<212>  PRT
<213>  Artificial Sequence

<220>
<223>  CDR-L1

<400>  1

Arg Ser Ser Gln Ser Ile Val His Ser Asn Gly Asn Thr Tyr Leu Glu
1               5                   10                  15


<210>  2
<211>  7
<212>  PRT
<213>  Artificial Sequence

<220>
<223>  CDR-L2

<400>  2

Lys Val Ser Asn Arg Phe Ser
1               5


<210>  3
<211>  9
<212>  PRT
<213>  Artificial Sequence

<220>
<223>  CDR-L3

<400>  3

Phe Gln Gly Ser His Val Pro Trp Thr
1               5


<210>  4
<211>  10
<212>  PRT
<213>  Artificial Sequence

<220>
<223>  CDR-H1

<400>  4

Gly Tyr Ser Phe Thr Gly Tyr Phe Met Asn
1               5                   10

<210>  5
<211>  17
<212>  PRT
<213>  Artificial Sequence

<220>
<223>  CDR-H2

<400>  5

Arg Ile Asn Pro Tyr Asn Gly Asp Thr Phe Tyr Asn Gln Lys Phe Lys
1               5                   10                  15

Gly

<210>  6
<211>  11
<212>  PRT
<213>  Artificial Sequence

<220>
<223>  CDR-H3

<400>  6

Trp Asp Gly Ala Pro Thr Tyr Phe Phe Asp Tyr
1               5                   10